(19) Europäisches Patentamt
European Patent Office
Office européen des brevets

(11) Numéro de publication: **0 000 306**
**B1**

(12) **FASCICULE DE BREVET EUROPEEN**

(51) Int. Cl³: C 07 D 491/18,
A 61 K 31/435, //
C 07 D 311/14,
C 07 D 311/16,
C 07 D 311/12,
(C 07 D 491/18, 311/00),
C 07 D 221/00

(21) Numéro de dépôt: **78400025.9**

(22) Date de dépôt: **19.06.78**

# (54) Hexahydro benzopyrano (3,2-c) pyridines substituées, leur procédé de préparation et médicament les contenant

(30) Priorité: **24.06.77 FR 7719360**
**01.06.78 FR 7816376**

(43) Date de publication de la demande:
**10.01.79 Bulletin 79/01**

(45) Mention de la délivrance du brevet:
**28.05.80 Bulletin 80/11**

(84) Etats contractants désignés:
**BE CH DE FR GB LU NL SE**

(56) Documents cités:
**FR - M - 7143**

(73) Titulaire: **LIPHA, Lyonnaise Industrielle Pharmaceutique**
**115 avenue Lacassagne B.P. No 106 Lyon R.P.**
**F - 69212 Lyon Cedex (FR)**

(72) Inventeurs: **Briet, Philippe**
**216 avenue Félix Faure**
**F - 69003 Lyon (FR)**
**Berthelon, Jean-Jacques**
**133 Cours Albert Thomas**
**F - 69003 Lyon (FR)**
**Depin, Jean-Claude**
**113, Cours Gambetta**
**F - 69003 Lyon (FR)**

(74) Mandataire: **Bouton Neuvy, Liliane**
**L'Air liquide, Société Anonyme pour L'Etude et L'Exploitation des Procédés Georges Claude**
**75 Quai d'Orsay**
**F - 75321 Paris Cedex 7 (FR)**

**EP 0 000 306 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

# Hexahydro benzopyrano [3,2-C] pyridines substituées, leur procédé de préparation et médicament les contenant

La présente invention concerne des hexahydro benzopyrano [3,2-C] pyridines substituées.

Par la publication de A. Sammour et M. Alkady (Indian Journal of Chemistry vol. 12, p. 51—53), on sait condenser des cétones, comme la cyclohexanone sur des carbéthoxy-3 coumarines pour obtenir des 2-amino-2,3 cyclohexanchronan-4$\alpha$ carbamido acétique acide lactame.

Il a été trouvé des hexahydro benzopyrano [3,2-C] pyridines représentées par la formule:

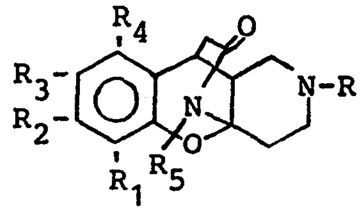

dans laquelle R est l'hydrogène ou un radical alcoyle inférieur saturé ou insaturé, linéaire ou ramifié, aralkyle, acyle, dialkylaminoalkyle, alkylcarbonyle, alcoxycarbonyle, halogénoalcoxycarbonyle, aryle, benzyloxycarbonyle, éthoxalyle et éthoxycarbonylméthyle; $R_1$ est l'hydrogène, un halogène ou un radical alcoxy inférieur; $R_2$ est l'hydrogène ou un halogène; $R_3$ est l'hydrogène, un halogène, un radical alcoyle inférieur, alcoxy, nitro, amino ou forme le naphtalène avec $R_4$ et le cycle benzénique; $R_4$ est l'hydrogène, un halogène ou forme le naphtalène avec $R_3$ et le cycle benzénique; $R_5$ est l'hydrogène, un radical alcoyle inférieur ou arylalkyle.

Les sels de ces nouveaux composés avec les acides minéraux et organiques acceptables en thérapeutique humaine font partie de l'invention. Ces composés possèdent de remarquables propriétés pharmacologiques qui les rendent intéressants en médecine humaine notamment pour le traitement des états dépressifs et des troubles psychiques.

La structure polycyclique des composés laisse prévoir pour un composé donné l'existence de plusieurs stéréoisomères. L'examen en chromatographie sur couche mince des produits de réaction fait apparaitre l'existence de deux stéréoisomères A et B. Une recristallisation dans un solvant adéquate est suffisante pour isoler à l'état pur le stéréoisomère majoritaire du produit brut réactionnel. Le stéréoisomère minoritaire peut s'obtenir par concentration des eaux-mères. Il est possible de transformer le stéréoisomère minoritaire par un traitement acide en le stéréoisomère majoritaire.

On a mis en évidence pour certains composés l'existence des deux stéréoisomères A et B. En conséquence, on met en oeuvre deux modes de synthèse de ces composés permettant d'accéder soit à la série A, soit à la série B.

Les procédés de préparation sont illustrés dans la séquence suivante où R, $R_1$, $R_2$, $R_3$, $R_4$ et $R_5$ ont les significations précédemment données.

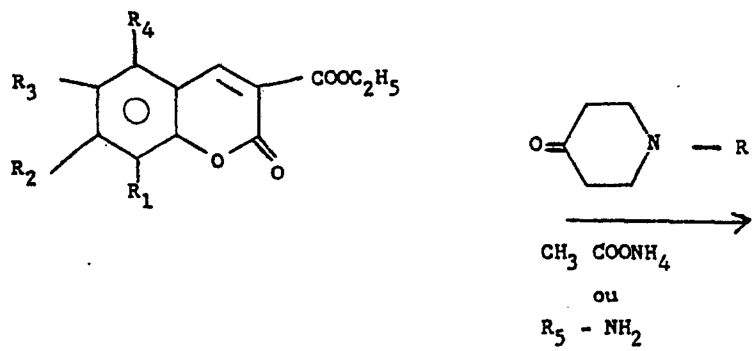

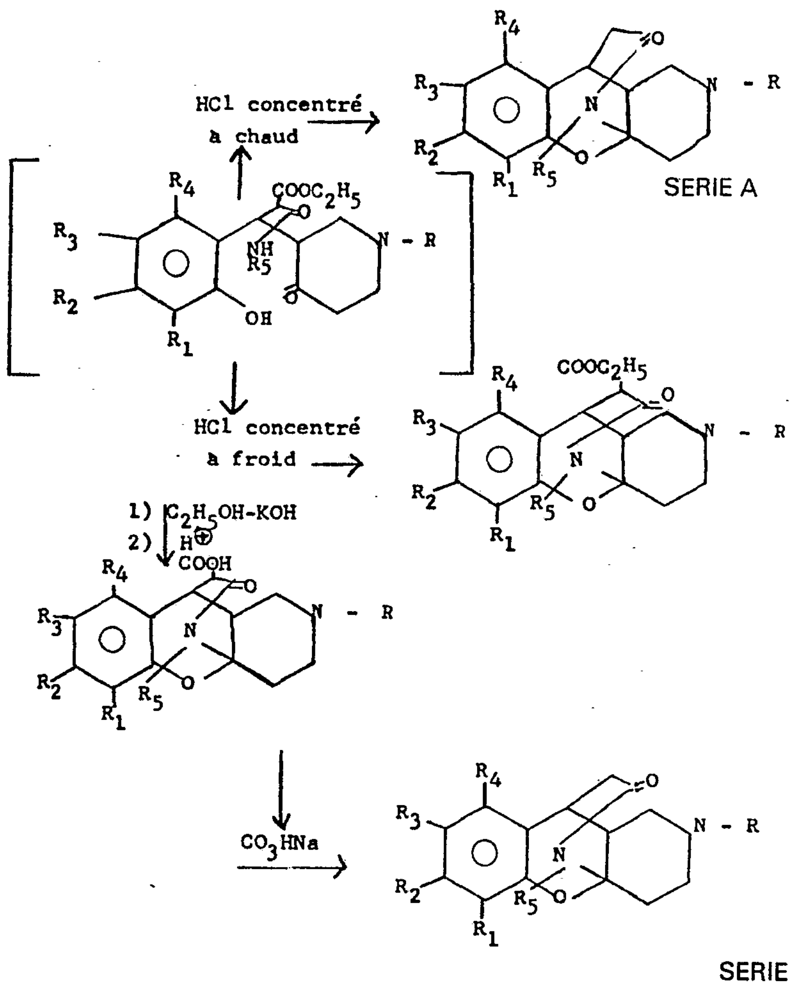


L'addition selon Michaêl d'un pipéridone-4N-substituée sur une coumarine carboxylate d'éthyle-3 et l'ouverture de l'adduct résultant par l'acétate d'ammonium générateur d'ammoniac ou par une amine primaire $R_5$—$NH_2$ s'effectue en chauffant les composés ensemble à des températures comprises entre 20 et 200°C en présence ou non d'un solvant alcoolique pendant un temps variant de 3 à 70 heures. Un traitement par l'acide chlorhydrique concentré à l'ébullition permet par cyclisation déshydratante d'accéder à la série Ⓐ des composés. Par contre un traitement par l'acide chlorhydrique concentré à froid permet l'isolement d'un ester $\beta$-cétonique hydrolysable par le couple potasse-alcool en acide correspondant.

La décarboxylation par chauffage dans le carbonate acide de sodium permet d'isoler les composés appartenant à la série Ⓑ. Le passage des composés de la série Ⓑ aux composés correspondants de la série Ⓐ est réalisable en traitant par l'acide chlorhydrique à chaud le dit composé de la série Ⓑ.

Selon une variante de procédé, quand R est l'hydrogène on peut traiter par hydrogénation catalytique un composé précédemment obtenu en particulier R = benzyle, puis substituer l'hydrogène par un radical alcoyle saturé ou insaturé, linéaire ou ramifié, aralkyle, dialkylaminoalkyle.

Selon une autre variante, on peut traiter un composé où R est alkyle tel que méthyle ou aralkyle tel que benzyle par un chlorocarbamate à l'ébullition dans un solvant aromatique et isoler ainsi les composés où R est un alcoxycarbonyle

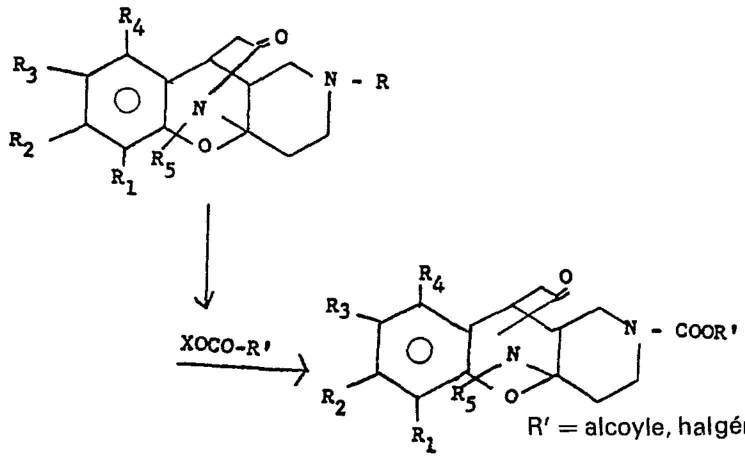


R' = alcoyle, halgénoalcoyle, aralkyle

En particulier quand $R_1$ ou $R_2$ ou $R_3$ ou $R_4$ est un halogène il est avantageux de traiter un composé où R = benzyle par le chloroformiate de benzyle et d'hydrolyser le carbonate obtenu par l'acide bromhydrique pour pouvoir accéder aux composés où R est H quand $R_1$ ou $R_2$ ou $R_3$ ou $R_4$ est un halogène. Il est ensuite commode de substituer cet hydrogène par un radical alcoyle saturé ou insaturé, linéaire ou ramifié, aralkyle, dialkylaminoalkyle

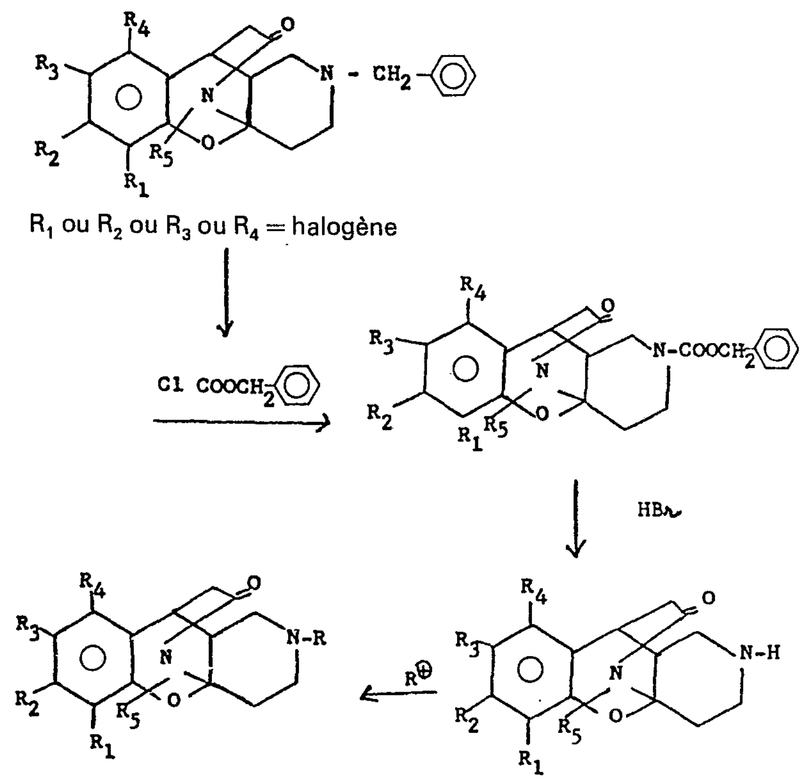


Les halogéno coumarine carboxylate d'éthyle-3, intermédiaires de synthèse tels les fluoro-6, chloro-5 et chloro-7 coumarine carboxylate d'éthyle-3 sont nouvelles.

Les nouvelles hexahydro benzopyrano [3,2-C] pyridines substituées possèdent de remarquables propriétés qui en font des médicaments utiles dans les états dépressifs et les troubles psychiques. Cette activité modificatrice de l'humeur peut être déterminée par des tests normalisés bien connus des spécialistes. Ainsi, les composés de l'invention se sont révélés être de puissants inhibiteurs du ptosis à la réserpine.

Sur la souris Swiss, on donne un composé P.O. simultanément avec la réserpine I.P. à 5 mg/kg. Le ptosis, coté selon Rubin B. and Coll. (J. Pharmacol. Exp. Therap., 1957, 120—125) à 1h, 1h30 et 2h plus tard permet de déterminer la dose inhibant le ptosis moyen de 50%. On consigne dans le tableau I les doses efficaces 50 (DE.50) obtenues pour quelques produits et celles obtenues pour des substances étalons bien connues des spécialistes, telles l'Imipramine [chlorhydrate de N-(3-diméthylaminopropyl) iminodibenzyle] et l'Amitripthyline [chlorhydrate de (diméthylamino-3) propylidène)-5 dibenzo (a,d) cycloheptadiène-1,4].

TABLEAU I

| Produits | DE.50 en mg/kg |
|---|---|
| Imipramine | 2,9 |
| Amitripthyline | 10 |
| Exemple 1A | 6 |
| Exemple 1B | 24 |
| Exemple 5A | 0,04 |
| Exemple 5B | 11 |
| Exemple 6 | 0,1 |
| Exemple 7 | 3,5 |
| Exemple 8 | 1 |
| Exemple 9 | 5 |
| Exemple 10 | 1,8 |
| Exemple 15 | 0,6 |
| Exemple 18 | 0,67 |
| Exemple 27 | 0.37 |
| Exemple 28 | 0,25 |
| Exemple 36 | 0,7 |

On évalue les toxicités sur la souris Swiss. Les doses léthales 50 (DL 50) par voie orale sont données dans le tableau suivant:

TABLEAU II

| Produits | DL 50 P.O. mg/kg |
|---|---|
| Imipramine | 330 |
| Amitripthyline | 150 |
| Exemple 1.A. | 2700 |
| Exemple 1.B. | 600 |
| Exemple 5.A. | 360 |
| Exemple 5.B. | 2000 |
| Exemple 6 | 720 |
| Exemple 7 | 1200 |
| Exemple 8 | 1080 |
| Exemple 9 | >3200 |
| Exemple 10 | 600 |
| Exemple 15 | 2470 |
| Exemple 18 | 1200 |
| Exemple 27 | >1600 |
| Exemple 28 | 1100 |
| Exemple 36 | 600 |

Le médicament contenant comme principe actif un composé de l'invention, associé à un véhicule ou excipient pharmaceutique acceptable, est présenté sous une forme adaptée en vue d'une administration orale ou parentérale.

Les formes posologiques sont unitaires telles que des comprimés, des capsules, des gélules, des ampoules ... Ces formes posologiques renferment de 0,05 à 100 mg de substance active et permettent une administration quotidienne de 1 à 200 mg.

EXEMPLES

— *Composition d'un comprimé de 100 mg éventuellement enrobé:*

| | |
|---|---|
| Principe actif | 5 mg |
| Lactose | 41 mg |
| Amidon de blé | 41 mg |
| Gélatine | 2 mg |
| Acide alginique | 5 mg |
| Talc | 5 mg |
| Stéarate de magnésium | 1 mg |

— *Composition d'une gélule:*

| | |
|---|---|
| Principe actif | 2 mg |
| Lactose | 30 mg |
| Amidon de blé | 35 mg |
| Talc | 2,5 mg |
| Stéarate de magnésium | 0,5 mg |

— *Composition d'une solution injectable:*

| | |
|---|---|
| Principe actif | 5 mg |
| Chlorure de sodium | 18 mg |
| Eau pour préparation injectable q.s.p. | 2 ml |

Parmi les observations recueillies en essai clinique du médicament contenant le principe actif de l'exemple 5.A.

OBSERVATION N° 1

*Nom:* DAC... Agnès *Age:* 78 ans *Sexe:* F
*Diagnostic:* Dépression pré-sénile.
*Traitements associés:* NOZINAN-SUREPTIL.
*Posologie et durée du traitement:* 5 mg/jour pendant 30 jours.
*Activité:* Reprise du poids. Amélioration de l'humeur et de l'insertion sociale.
*Tolérance*
. clinique
. biologique } R.A.S.

OBSERVATION N° 2

*Nom:* LAR... Gineste *Age:* 58 ans *Sexe:* M
*Diagnostic:* Dépression post-neuroleptique chez un schizophrène.
*Traitements associés:* NOZINAN-ARTANE-GARDENAL.
*Posologie et durée du traitement:* 20 mg/jour pendant 30 jours
*Activité:* Augmentation du poids. Amélioration de l'humeur et du sommeil. Diminution de l'anxiété. Amélioration des troubles hypocondriaques.
*Tolérance:*
. clinique
. biologique } R.A.S.

OBSERVATION N° 3

*Nom:* JAC... Marcel *Age:* 55 ans *Sexe:* M
*Diagnostic:* Syndrome dépressif hypocondriaque grave
*Traitements associés:* MEPRONIZINE.
*Posologie et durée du traitement:* 10 mg/jour pendant 20 jours
*Activité:* Le malade est moins préocupé de son corps. Diminution des rites de lavage. Amélioration du comportement social.
*Tolérance:*
. clinique
. biologique } R.A.S.

OBSERVATION N°4

*Nom:* MIC... Louise *Age:* 55 ans *Sexe:* F
*Diagnostic:* Rechute dépressive de type mélancolique.
*Traitements associés:* NOZINAN 25 mg.
*Posologie et durée du traitement:* 25 mg/jour pendant 45 jours
*Activité:* Amélioration de l'humeur. Attitude moins pessimiste. Pleurs moins fréquents. Sentiment de culpabilité moins prononcé.
*Tolérance:*
. clinique
. biologique } R.A.S.

OBSERVATION N° 5

*Nom:* TIL... François *Age:* 47 ans *Sexe:* M
*Diagnostic:* Syndrome dépressif chez un éthylique de type mélancolique.
*Traitements associés:* Perfusions de polyvitamines — NOZINAN 50 mg — ARTANE 5.
*Posologie et durée du traitement:* 5 mg/jour pendant 30 jours.
*Activité:* Amélioration de l'humeur. Pleurs moins fréquents. Augmentation de l'appétit.
*Tolérance:*
. clinique: quelques vertiges et nausées
. biologiques: R.A.S.

OBSERVATION N° 6

*Nom:* BEL... André *Age:* 45 ans *Sexe:* M
*Diagnostic:* Délire hypocondriaque avec anxiété.
*Traitements associés:* ARTANE 10 mg — ANTASTHENE.
*Posologie et durée du traitement:* 25 mg/jour pendant 30 jours.
*Activité:* Diminution des troubles phypocondriaques. Malade moins irritable et craintif.
*Tolérance:*
. clinique: quelques vertiges;
. biologique: R.A.S.

Il est donné ci-après des exemples de préparation des composés de l'invention.

EXEMPLE 1

*Lactame de l'acide [amino-4a méthyl-2 hexahydro-1,2,3,4,4a,10a[10H](benzopyrano)(3,2-C)(pyridine)]-yl-10, acétique* (formule 1), $C_{15}H_{18}N_2O_2$ P.M. = 258,31

On solubilise dans 34 litres d'éthanol anhydre, 2,182 kg (10 moles) de coumarine-3 carboxylate d'éthyle, 1,144 kg (10 moles) de N-méthyl 4-pipéridone, on ajoute 1,544 kg (20 moles) d'acétate d'ammonium et on agite le milieu réactionnel 72 heures à température ambiante. On port une heure à reflux et on évapore environ 25 litres d'éthanol. On reprend la masse résineuse par 8,9 litres d'acide chlorhydrique concentré et on porte une demi-heure à reflux. On refroidit le milieu réactionnel au moyen d'un bain de glace et on amène à pH alcalin avec 9 litres de NaOH 30%. On essore le solide obtenu qui se présente sous la forme d'un produit crème fondant à 228°C. On recristallise dans 10 litres d'isopropanol. On obtient 2,083 kg (Rdt 70%) de lactame de l'acide [amino-4a, méthyl-2 hexahydro-1,2,3,4a,10a[10H](benzopyrano)(3,2-C)(pyridine)]-yl-10-acétique (produit A) $PF_G = 224°C$, $IR_{\nu C=O}$: 1690 $cm^{-1}$.

*Analyse:*

| | C % | H % | N % | O % |
|---|---|---|---|---|
| calculée | 69,74 | 7,02 | 10,85 | 12,39 |
| trouvée | 69,62 | 6,97 | 10,85 | |

*Chlorhydrate:* $PF_G = 250—252°C$ (éthanol).
*C.G.M.* plaque alcaline 0,1 N gel silice, éluant: méthanol-chloroforme—cyclohexane (1—3—5): 1 spot. Par concentration des eaux-mères on obtient un produit B: $PF_G = 232—234°C$, $IR_{\nu C=O}$: 1680 $cm^{-1}$.

*Analyse:*

| | C % | H % | N % | O % |
|---|---|---|---|---|
| calculée | 69,74 | 7,02 | 10,85 | 12,39 |
| trouvée | 69,72 | 6,90 | 10,70 | |

Passage de B à A: On porte à reflux pendant une heure, 10 grammes de B dans 100 ml d'acide chlorhydrique concentré. Après refroidissement, on basifie avec NaOH 30%, on extrait avec $CH_2Cl_2$, on sèche sur $Na_2SO_4$, on évapore le solvant. On obtient un solide blanc dont les caractéristiques physicochimiques sont en tous points identiques à celles de A.

EXEMPLE 2

*Lactame de l'acide [amino-4a benzyl-2 hexahydro-1,2,3,4,4a,10a[10H](benzopyrano)(3,2-C)(pyridine)]-yl-10 acétique* (formule 2) $C_{21}H_{22}N_2O_2$, P.M. = 334,42

Préparé selon l'exemple 1, à partir de 47,6 g (0,218 mole) de coumarine-3 carboxylate d'éthyle, 33,4 g (0,218 mole) de N-benzyl 4-pipéridone, 34,8 g (0,436 mole) d'acétate d'ammonium, dans 1,9 litre d'éthanol anhydre. On obtient 43,7 g (Rdt = 60%) de lactame de l'acide amino-4a, benzyl-2 hexahydro-1,2,3,4,10a[10H](benzopyrano)(3,2-C)pyridine]yl-10 acétique sous forme d'un solide blanc. $PF_G = 192°C$ (éthanol).
*Chlorhydrate:* $C_{21}H_{23}ClN_2O_2$, P.M. = 370,87, $PF_G = 254—255°C$ (méthanol) $IR_{\nu C=O}$: 1680 $cm^{-1}$.

*Analyse:*

| | C % | H % | N % | Cl % | O % |
|---|---|---|---|---|---|
| calculée | 68 | 6,25 | 7,55 | 9,56 | 8,63 |
| trouvée | 68,32 | 6,53 | 7,84 | 9,61 | |

## EXEMPLE 3

*Lactame de l'acide [amino-4a méthyl-2 méthoxy-6 hexahydro-1,2,3,4,4a,10a[10H](benzopyrano)(3,2-C)-(pyridine)]yl-10 acétique* (formule 3) $C_{16}H_{20}N_2O_3$, P.M. = 288,35

Préparé selon l'exemple 1 à partir de 93 g (0,375 mole) de méthoxy-8 carbéthoxy-3 coumarine, 30,6 g (0,375 mole) de N-méthyl 4-pipéridone, 41,4 g (0,75 mole) d'acétate d'ammonium et 3,6 litres d'alcolle. On obtient 48,6 g (Rdt = 45%) de lactame de l'acide [amino-4a méthyl-2 méthoxy-6 hexahydro-1,2,3,4,4a,10a, [10H](benzopyrano)(3,2-C)(pyridine)]yl-10 acétique sous forme d'un solide blanc, $PF_G = 258°C$.

*Chlorhydrate, monohydrate:* $C_{16}H_{23}ClN_2O_4$ P.M. = 342,82 $PF_G$ = 265°C (methanol) $IR_{\nu C=O}$: 1680 $cm^{-1}$ $IR_{\nu -OH}$.

*Analyse:*

| | C % | N % | Cl % | N % | O % |
|---|---|---|---|---|---|
| calculée | 56,04 | 6,76 | 10,34 | 8,17 | 18,66 |
| trouvée | 56,23 | 6,39 | 10,60 | 8,09 | |

## EXEMPLE 4

*Lactame de l'acide [amino-4a hexahydro-1,2,3,4,4a,10a, [10H](benzopyrano)(3,2-C)(pyridine)]-yl-10 acétique* (formule 4) $C_{14}H_{16}N_2O_2$, P.M. = 244,30

On solubilise 34,3 g du produit de l'exemple 2 dans 320 ml d'éthanol et on porte 5 heures à 60°C, sous une pression initiale d'hydrogène de 60 kg, en présence de 4,7 g de Pd/C 10%.

Après refroidissement, filtration du catalyseur et évaporation du solvant on obtient le lactame de l'acide [amino-4a hexahydro-1,2,3,4,4a,10a, [10H](benzopyrano)(3,2-C)(pyridine)]yl-10 acétique sous forme d'un solide blanc fondant à 212°C. Poids obtenu = 20 g (Rdt: 80%) $IR_{\nu C=O}$: 1680 $cm^{-1}$.

*Chlorhydrate* $C_{14}H_{17}ClN_2O_2$, P.M. = 280,76 $PF_G$ = 292—294°C (méthanol).

*Analyse:*

| | C % | H % | N % | Cl % | O % |
|---|---|---|---|---|---|
| calculée | 59,89 | 6,10 | 9,98 | 12,63 | 11,40 |
| trouvée | 59,71 | 6,18 | 9,87 | 12,38 | |

## EXEMPLE 5

*Lactame de l'acide [amino-4a chloro-8 méthyl-2 hexahydro-1,2,3,4,4a,10a, [10H](benzopyrano)-(3,2-C)(pyridine)]yl-10 acétique* (formule 5) $C_{15}H_{17}ClN_2O_2$ P.M. = 292,77

5.1 *Produit A*

On porte à reflux pendant 8 heures 101,06 g (0,4 mole) de chloro-6 coumarine-3 carboxylate d'éthyle, 45,76 g (0,4 mole) de N-méthyl pipéridone-4, 61,7 g (0,8 mole) d'acétate d'ammonium et 12 litres d'éthanol. On évapore le solvant et le résidu est repris par 320 ml d'acide chlorhydrique concentré et la solution obtenue est portée une demi-heure à reflux. On alcalinise ensuite avec NaOH 30%, en refroidissant le milieu par un bain de glace. On dilue ensuite avec $H_2O$ et on extrait au chloroforme. Après séchage sur $Na_2SO_4$ et évaporation on obtient 148 g d'un solide beige. On recristallise dans le mélange acétone-méthanol. On obtient 55 g de lactame de l'acide [amino-4a chloro-8 méthyl-2 hexahydro-1,2,3,4,4a,10a [10H](benzopyrano)(3,2-C)(pyridine)]yl-10 (Rdt: 47%) $PF_G$ = 245°C $IR_{\nu C=O}$: 1680 $cm^{-1}$.

*Analyse:*

| | C % | H % | Cl % | N % | O % |
|---|---|---|---|---|---|
| calculée | 61,53 | 5,85 | 12,11 | 9,57 | 10,93 |
| trouvée | 61,76 | 5,83 | 12,01 | 9,56 | |

*Chlorhydrate:* $C_{15}H_{18}Cl_2N_2O_2$ P.M. = 329,22 $PF_G$ = 260—262°C (éthanol).

*Méthanesulfonate:* On solubilise 10 g du produit de l'exemple 5.1 dans la quantité nécessaire de chloroforme à 60°C. On refroidit à 41°C et on ajoute 2,5 ml d'acide méthane sulfonique en solution dans 7,5 ml de chloroforme. Par refroidissement on obtient un produit blanc que l'on essore et que l'on recristallise dans le méthanol $PF_G$ = 265—267°C.

*Analyse:*

| | C % | H % | Cl % | N % | O % | S % |
|---|---|---|---|---|---|---|
| calculée | 49,42 | 5,44 | 9,12 | 7,20 | 20,57 | 8,24 |
| trouvée | 49,29 | 5,45 | 8,95 | 7,13 | | |

5.2 *Produit B*

*5.2.1.:* Lactame du monoester éthylique de l'acide [amino-4a, chloro-8 méthyl-2 hexahydro-1,2,3,4,4a,10a, [10H](benzopyrano)(3,2-C)(pyridine)]yl-10 malonique $C_{18}H_{21}ClN_2O_4$ PM = 364,827.

On porte à reflux pendant 8 heures 80 g (0,31 mole) de chloro-6 coumarine-3 carboxylate d'éthyle 35,8 g (0,31 mole) de N-méthyl pipéridone-4, 48,8 g (0,62 mole) d'acétate d'ammonium et 1240 ml d'éthanol. Après évaporation on reprend le résidu par 600 ml d'acide chlorohydrique concentré, à froid, et on agite 1 heure. On ajuste à pH alcalin par addition de 600 ml de soude 30% et 600 g de glace en maintenant la température en dessous de 25°C. On extrait avec 2 fois 1 litre de chloroforme. On sèche la phase organique sur sulfate de sodium, on évapore. Par recristallisation dans l'éthanol du résidu obtenu, on isole 63 g du produit attendu avec un rendement de 56% $PF_G$ = 190°C.

*5.2.2.* Lactame de l'acide [amino-4a, chloro-8, méthyl-2 hexahydro-1,2,3,4,4a,10a, [10H]-(benzopyrano)(3,2-C)(pyridine)]-yl-10 malonique.

A une suspension de 53 g (0,15 mole) du produit de l'exemple 5.2.1 dans 400 ml d'éthanol on ajoute une solution de 21 g (0,375 mole) de potasse dans 400 ml d'eau. On porte ensuite 1 heure à reflux. Puis on refroidit à 12°C et on ajuste à pH 5—6 par addition d'acide chlorhydrique N. On laisse reposer une nuit et on essore le précipité. On obtient 35 g du produit attendu avec un rendement de 75, 5% PF = 210—215°C avec décomposition.

5.2.3. *Produit B*

A une solution de 2,5 g (0,03 mole) de bicarbonate de sodium dans 100 ml d'eau, on ajoute 10 g (0,03 mole) du produit de l'exemple 5.2.2 et on porte au reflux 1 heure. Un précipité se forme progressivement. On refroidit ensuite et on essore. Par recristallisation dans l'alcool éthylique on obtient 4,1 g de Produit B $PF_G$ = 246°C $IR_{\nu C=O}$: 1700 $cm^{-1}$.

*Analyse:*

| | C % | H % | N % |
|---|---|---|---|
| calculée | 61,53 | 5,83 | 9,57 |
| trouvée | 61,36 | 5,85 | 9,63 |

EXEMPLE 6

*Lactame de l'acide [amino-4a bromo-8 méthyl-2 hexahydro-1,2,3,4,4a,10a, [10H](benzopyrano)-(3,2-C)(pyridine)]yl-10 acétique* (formule 6) $C_{15}H_{17}BrN_2O_2$ P.M. = 337,23

Préparé selon l'exemple 5.1 à partir de 47 g (0,158 mole) de bromo-6 coumarine-3 carboxylate d'éthyle, 18,1 g (0,158 mole) de N-méthyl pipéridone-4, 24,4 g (0,316 mole) d'acétate d'ammonium et 3 litres d'éthanol. On obtient après recristallisation dans le mélange acétone-éthanol, 26 g de lactame de l'acide [amino-4a bromo-8 méthyl-2 hexahydro-1,2,3,4,4a,10a, [10H](benzopyrano)(3,2-C)-(pyridine)]yl-10 acétique. (Rdt =49%) $PF_G$ = 237—239°C $IR_{\nu C=O}$: 1690 $cm^{-1}$.

*Analyse:*

| | C % | N % | Br % | N % | O % |
|---|---|---|---|---|---|
| calculée | 53,42 | 5,08 | 23,70 | 8,31 | 9,49 |
| trouvée | 53,60 | 5,08 | 23,87 | 8,40 | |

EXEMPLE 7

*Lactame de l'acide [amino-4a diméthyl-2,8 hexahydro-1,2,3,4,4a,10a, [10H](benzopyrano)(3,2-C)-(pyridine)]yl-10 acétique* (formule 7) $C_{16}H_{20}N_2O_2$ P.M. = 272,34

Préparé selon l'exemple 5.1 à partir de 60 g (0,258 mole) de méthyl-6 coumarine-3 carboxylate d'éthyle, 29,6 g (0,258 mole) de N-méthyl pipéridone-4, 39,9 g (0,516 mole) d'acétate d'ammonium et 2 litres d'éthanol. Après recristallisation dans le mélange acétone-éthanol, on obtient 39,3 g de lactame de l'acide [amino-4a diméthyl-2,8 hexahydro-1,2,3,4,4a,10a[10H](benzopyrano)(3,2-C)(pyridine)]yl-10 acétique (Rdt = 56%) $PF_G$ = 241—243°C $IR_{\nu C=O}$: 1680 $cm^{-1}$.

*Analyse:*

| | C % | H % | N % | O % |
|---|---|---|---|---|
| calculée | 70,56 | 7,40 | 10,29 | 11,75 |
| trouvée | 70,58 | 7,41 | 10,38 | |

EXEMPLE 8

*Lactame de l'acide [amino-4a fluoro-8 méthyl-2 hexahydro-1,2,3,4,4a,10a, [10H](benzopyrano)-(3,2-C)(pyridine)]yl-10 acétique* (formule 9) $C_{15}H_{17}FN_2O_2$ P.M. = 276,31

8.1 *-Fluoro-6 coumarine-3 carboxylate d'éthyle* (formule 8) $C_{12}H_9FO_4$ P.M. = 236,19.

On porte 3 heures à reflux 77 g (0,55 mole) de fluoro-4 hydroxy-2 benzaldéhyde, 96,6 g (0,604 mole) de malonate d'éthyle, 220 ml d'éthanol, 2,9 ml de pipéridine et 0,3 ml d'acide acétique glacial.

On verse dans 600 ml d'eau glacée. On filtre et on recristallise dans l'éthanol le fluoro-6 coumarine-3 carboxylate d'éthyle, $PF_G = 108°C$, $IR_{\nu C=O}$: 1710 $cm^{-1}$ (lactone), 1730 $cm^{-1}$ (ester).

*RMN* ($CDCl_3$) $\delta$ ppm par rapport au TMS
3H à 1,4 (triplet)
2H à 4,35 (quartet)
3H de 7,2 à 7,6 (massif)
1H à 8,5 (singulet)

8.2 — *Lactame de l'acide [amino-4a fluoro-8 méthyl-2 hexahydro-1,2,3,4,4a,10a, [10H](benzopyrano)(3,2-C)(pyridine)]-yl-10 acétique* (formule 9) $C_{15}H_{17}FN_2O_2$ P.M. = 276,31

Préparé selon l'exemple 5.1 à partir de 80 g (0,339 mole) de fluoro-6 coumarine-3 carboxylate d'éthyle, 38,8 g (0.339 mole) de N-méthyl pipéridone-4, 52,4 g (0,678 mole) d'acétate d'ammonium et 2 litres d'éthanol. On obtient, après recristallisation dans l'isopropanol 37,5 g de lactame de l'acide [amino-4a fluoro-8 méthyl-2 hexahydro-1,2,3,4,4a,10a, [10H](benzopyrano)(3,2-C)(pyridine)]-yl-10 acétique (Rdt = 40%) $PF_G$ = 226—228°C $IR_{\nu C=O}$: 1680 $cm^{-1}$

*Analyse:*

| | C % | H % | F % | N % | O % |
|---|---|---|---|---|---|
| calculée | 65,20 | 6,20 | 6,88 | 10,40 | 11,58 |
| trouvée | 64,83 | 6,09 | 7,20 | 10,49 | |

## EXEMPLE 9

*Lactame de l'acide [amino-4a acétyl-2 hexahydro-1,2,4,4a,10a, [10H](benzopyrano)(3,2-C)(pyridine)]-yl-10 acétique* (formule 10) $C_{16}H_{18}N_2O_3$ P.M. = 286,32

On solubilise 12,2 g (0,05 mole) du produit de l'exemple 4 dans 200 ml de chloroforme. On ajoute 14 ml (0,1 mole) de triéthylamine et on additionne goutte à goutte à 20°C 4,3 ml (0,06 mole) de chlorure d'acétyle. On laisse sous agitation 6 heures à température ambiante. On lave avec 400 ml d'eau, on sèche sur sulfate, on évapore et recristallise dans un mélange méthanol-chloroforme. On obtient, 7,1 g de lactame de l'acide [amino-4a acétyl-2 hexahydro-1,2,3,4,4a,10a, [10H](benzopyrano)(3,2-C)(pyridine)]-yl-10 acétique (Rdt = 50%) $PF_G$ = 284—287°C $IR_{\nu C=O}$: 1680 $cm^{-1}$.

*Analyse:*

| | C % | H % | N % | O % |
|---|---|---|---|---|
| calculée | 67,11 | 6,33 | 9,78 | 16,76 |
| trouvée | 66,82 | 6,05 | 9,98 | |

## EXEMPLE 10

*Lactame de l'acide [amino-7a méthyl-10 hexahydro-7a,8,9,10,11,11a, [12H](benzo)(f)(benzopyrano)-(3,2-C)pyridine]-yl-10 acétique* (formule 11) $C_{19}H_{20}N_2O_2$, P.M. = 308,39

Préparé selon l'exemple 5.1 à partir de 70 g (0,261 mole) de benzo (f) coumarine-3 carboxylate d'éthyle, 30 g (0,261 mole) de N-méthyl pipéridone-4, 40,4 g (0,522 mole) d'acétate d'ammonium, et 1500 ml d'éthanol. Après recristallisation dans le mélange acétate d'éthyle-méthanol, on obtient 44,2 g de lactame de l'acide [amino-7a méthyl-10 hexahydro-7a,8,9,10,11,11a, [12H](benzo)(f)-*(benzopyrano)(3,2-C)pyridine]yl-10 (Rdt* = 55%) $PF_G$ = 263—265°C $IR_{\nu C=O}$: 1680 $cm^{-1}$.

*Analyse:*

| | C % | H % | N % | O % |
|---|---|---|---|---|
| calculée | 74,00 | 6,54 | 9,09 | 10,37 |
| trouvée | 73,91 | 6,46 | 8,96 | |

## EXEMPLE 11

*Lactame de l'acide [amino-4a n-propyl-2 hexahydro-1,2,3,4,4a,10a [10H](benzopyrano)(3,2-C)-(pyridine)]-yl-10 acétique* (formule 12) $C_{17}H_{22}N_2O_2$, P.M. = 286,38

On porte 10 heures à 80°C une solution de 15 g (0,061 mole) du produit de l'exemple 4 dans 300 ml de diméthylformamide avec 7,9 g (0,064 mole) de bromure de propyle et 9,1 g (0,66 mole) de carbonate de potassium. On filtre l'insoluble, on évapore le solvent sous vide. On purifie dans l'acétate d'éthyle. On obtient 9,9 g de lactame de l'acide [amino-4a n-propyl-2 hexahydro-1,2,3,4,4a,10a, [10H](benzopyrano)(3,2-C)pyridine)]yl-10 acétique (Rdt = 56,7%) $PF_G$ = 182—184°C $IR_{\nu C=O}$: 1680 $cm^{-1}$.

*Analyse:*

| | C % | H % | N % | O % |
|---|---|---|---|---|
| calculée | 71,30 | 7,74 | 9,78 | 11,17 |
| trouvée | 71,02 | 7,59 | 9,66 | |

EXEMPLE 12

*Lactame de l'acide [amino-4a (phénylpropyl)-2 hexahydro-1,2,3,4,4a,10a, [10H](benzopyrano)(3,2-C)-(pyridine)]-yl-10 acétique* (formule 13) $C_{23}H_{26}N_2O_2$ P.M. = 362,47

Préparé selon l'exemple 11 à partir du produit de l'exemple 4 (15 g, 0,061 mole), 12,8 g (0,064 mole) de bromure de phényl-propyle, on obtient 12,1 g de lactame de l'acide [amino-4a (phénylpropyl)-2 hexahydro-1,2,3,4,4a,10a [10H](benzopyrano)(3,2-C)(pyridine)]yl-10 acétique (Rdt = 54,7%) $PF_G$ = 154—156°C (acétate d'éthyle) $IR_{\nu C=O}$: 1690 $cm^{-1}$.

*Analyse:*

| | C % | H % | N % | O % |
|---|---|---|---|---|
| calculée | 76,22 | 7,23 | 7,73 | 8,83 |
| trouvée | 75,97 | 7,01 | 7,62 | |

EXEMPLE 13

*Lactame de l'acide [amino-4a diméthylaminopropyl-2 hexahydro-1,2,3,4,4a,10a, [10H](benzopyrano)-(3,2-C)(pyridine)]-yl-10 acétique* (formule 14) $C_{19}H_{27}N_3O_2$ P.M. = 329,44

Préparé selon l'exemple 11 à partir du produit de l'exemple 4, 12,1 g (0,049 mole), 6,3 g (0,0516 mole) de chloro-1 diméthylamino-2 propane. On obtient 7,6 g de lactame de l'acide [amino-4a dimethylaminopropyl-2 hexahydro-1,2,3,4,4a,10a [10H](benzopyrano)(3,2-C)(pyridine)]yl-10 acétique. (Rdt = 47%) $PF_G$ = 149—150°C (acétate d'éthyle), $IR_{\nu C=O}$: 1680 $cm^{-1}$.

*Analyse:*

| | C % | H % | N % | O % |
|---|---|---|---|---|
| calculée | 69,27 | 8,26 | 12,75 | 9,75 |
| trouvée | 68,92 | 8,01 | 12,93 | |

EXEMPLE 14

*Lactame de l'acide [amino-4a chloro-7 méthyl-2 hexahydro-1,2,3,4,4a,10a, [10H](benzopyrano)-(3,2-C)(pyridine)]-yl-10 acétique* (formule 16) $C_{15}H_{17}ClN_2O_2$ P.M. = 292,77

14.1 *Chloro-7 coumarine-3 carboxylate d'éthyle* (formule 15) $C_{12}H_9ClO_4$ P.M. = 252,5

On porte 5 heures à reflux 11,2 g (0,071 mole) de chloro-4 hydroxy-2 benzaldéhyde, 12,5 g (0,072 mole) de malonate d'éthyle, 30 ml d'éthanol, 0,4 ml de pipéridine et 0,1 ml d'acide acétique. On refroidit le milieu réactionnel à 0°C et on essore le précipité formé. On lave à l'hexane et on sèche le chloro-7 coumarine-3 carboxylate d'éthyle obtenu $PF_G$ = 122—3°C, $IR_{\nu C=O}$: 1760 $cm^{-1}$ (lactone et ester).

*RMN* ($CDCl_3$) 5 p.p.m. par rapport au TMS

3H à 1,3 (triplet)

2H à 4,35 (quartet)

3H de 7,1 à 7,6 (massif)

1H à 8,45 singulet

14.2 *Lactame de l'acide [amino-4a, chloro-7 méthyl-2 hexahydro-1,2,3,4,4a,10a [10H](benzopyrano)(3,2-C)(pyridine)]-yl-10 acétique*

Préparé selon l'exemple 5.1 à partir de 6,5 g (0,026 mole) de chloro-7 coumarine-3 carboxylate d'éthyle, 2,9 g (0,026 mole) de N-méthylpipéridone-4, 4 g (0,052 mole) d'acétate d'ammonium et 150 ml d'éthanol. Après recristallisation dans l'éthanol on obtient 3 g du lactame attendu (Rdt = 40%). $PF_G$ = 256—258°C $IR_{\nu C=O}$: 1675 $cm^{-1}$.

*Analyse:*

| | C % | H % | Cl % | N % |
|---|---|---|---|---|
| calculée | 61,53 | 5,85 | 12,11 | 9,57 |
| trouvée | 61,34 | 5,86 | 12,40 | 9,63 |

EXEMPLE 15

*Lactame de l'acide [amino-4a, nitro-8 méthyl-2 hexahydro-1,2,3,4,4a,10a, [10H](benzopyrano)-(3,2-C)(pyridine)]-yl-10 acétique* (formule 17) $C_{15}H_{17}N_3O_4$ P.M. — 303,32

Préparé selon l'exemple 5.1 à partir de 70 g (0,27 mole) de nitro-6 coumarine-3 carboxylate d'éthyle, 30,6 g (0,27 mole) de N-méthyl-pipéridone-4, 41,3 g (0,54 mole) d'acétate d'ammonium et 1,5 litre d'éthanol. On obtient après recristallisation dans l'éthanol 38,8 g de lactame de l'acide [amino-4a, nitro-8-méthyyl-2 hexahydro-1,2,3,4,4a,10a [10H](benzopyrano)(3,2-C)(pyridine)]-yl-10 acétique (Rdt = 47,4%) $PF_G$ = 240—242°C $IR_{\nu C=O}$: 1680 $cm^{-1}$.

*Analyse:*

| | C % | H % | N % |
|---|---|---|---|
| calculée | 59,40 | 5,65 | 13,85 |
| trouvée | 59,25 | 5,66 | 13,74 |

EXEMPLE 16

*Lactame de l'acide [diamino-4a,8, méthyl-2, hexahydro-1,2,3,4,4a,10a, [10H](benzopyrano)(3,2-C)-(pyridine)]-yl-10 acétique* (formule 18) $C_{15}H_{19}N_3O_2$ P.M. = 273,34

On glace 19,9 g (0,065 mole) du produit de l'exemple 15 dans un autoclave avec 300 ml d'éthanol et 1 g de Pd/O à 10%. On charge l'autoclave sous une pression initiale en hydrogène de 60 kg/cm² et on laisse 3 heures à température ambiante, sous agitation, puis 2 heures à 50°C. Après refroidissement on filtre le palladium, on évapore et on recristallise le solide obtenu dans le mélange acétate d'éthyle-éthanol. On ambient 8,1 g de lactame de l'acide [diamino-4a,8, méthyl-2 hexahydro-1,2,3,4,4a,10a, [10H](benzopyrano)(3,2-C)(pyridine)]-yl-10 acétique (Rdt = 45,6%) $PF_G$ = 230—232°C $IR_{\nu C=O}$: 1680 $cm^{-1}$.

*Analyse:*

| | C % | H % | N % |
|---|---|---|---|
| calculée | 65,91 | 7,01 | 15,37 |
| trouvée | 65,77 | 6,88 | 15,22 |

EXEMPLE 17

*Lactame de l'acide [dichloro-6,8, amino-4a, méthyl-2, hexahydro-1,2,3,4,4a,10a, [10H](benzopyrano)-(3,2-C)(pyridine)]-yl-10 acétique* (formule 19) $C_{15}H_{16}Cl_2N_2O_2$ P.M. = 327,22

Préparé selon l'exemple 5.1 à partir de 17,8 g (0,062 mole) de dichloro-6,8, coumarine-3 carboxylate d'éthyle, 7,1 g (0,062 mole) de N-méthyl-pipéridone-4, 9,5 g (0,124 mole) d'acétate d'ammonium et 800 ml d'éthanol. On obtient, après recristallisation dans l'acétate d'éthyle 7,7 g de lactame de l'acide [dichloro-6,8, amino-4a, hexahydro-1,2,3,4,4a,10a, [10H](benzopyrano)(3,2-C)(pyridine)]-yl-10 acétique (Rdt = 38%) $PF_G$ = 212—216°C $IR_{\nu C=O}$: 1680 $cm^{-1}$.

*Analyse:*

| | C % | H % | Cl % | N % |
|---|---|---|---|---|
| calculée | 55,05 | 4,93 | 21,57 | 8,56 |
| trouvée | 54,89 | 4,96 | 21,53 | 8,51 |

EXEMPLE 18

*Lactame de l'acide [amino-4a, méthoxy-8, méthyl-2, hexahydro-1,2,3,4,4a,10a, [10H](benzopyrano)-(3,2-C)(pyridine)]-yl-10 acétique* (formule 20) $C_{16}H_{20}N_2O_3$ P.M. = 288,35

Préparé selon l'exemple 5.1 à partir de 35,5 g (0,143 mole) de méthoxy-6 coumarine-3 carboxylate d'éthyle, 16,4 g (0,143 mole) de N-méthyl-pipéridone-4, 22,1 g (0,286 mole) d'acétate d'ammonium et 400 ml d'éthanol. On obtient par recristallisation dans l'isopropanol 26 g de lactame de l'acide [amino-4a, méthoxy-8, méthyl-2, hexahydro-1,2,3,4,4a,10a, [10H](benzopyrano)(3,2-C)-(pyridine)]-yl-10 acétique (Rdt = 63%) $PF_G$ = 210—212°C $IR_{\nu C=O}$: 1680 $cm^{-1}$.

*Analyse:*

| | C % | H % | N % |
|---|---|---|---|
| calculée | 66,65 | 6,99 | 9,71 |
| trouvée | 66,61 | 6,89 | 9,67 |

EXEMPLE 19

*Lactame de l'acide [amino-4, chloro-9, méthyl-2, hexahydro-1,2,3,4,4a,10a, [10H](benzopyrano)-(3,2-C)(pyridine)]-yl-10 acétique* (formule 22) $C_{15}H_{17}Cl_2N_2O_2$ P.M. = 292,77

19.1 *Chloro-5 coumarine-3 carboxylate d'éthyle* (formule 21) $C_{12}H_9ClO_4$ P.M. = 252,5

On porte 5 heures à refiux 15 g (0,0958 mole) de chloro-2, hydroxy-6, benzaldéhyde, 16,7 g (0,105 mole) de malonate d'éthyle, 40 ml d'éthanol, 0,6 ml de pipéridine et 0,1 ml d'acide acétique. Après refroidissement on essore le produit obtenu. On sèche et on obtient 14 g de chloro-5, coumarine-3 carboxylate d'éthyle (Rdt = 58%) $PF_G$ =142—144°C.

$IR_{\nu C=O}$: 1720 $cm^{-1}$

$_{\nu C=O}$: 1760 $cm^{-1}$

*RMN* ($CDCl_3$) $\delta$ ppm par rapport au TMS

3H à 1,45 (triplet)

2H à 4,5 (quartet)

3H de 7,1 à 7,7 (massif)

1H à 8,8 (singulet)

19.2 *Lactame de l'acide [amino-4a, chloro-9, méthyl-2, hexahydro-1,2,3,4,4a,10a, [10H]-(benzopyrano)(3,2-C)(pyridine)]-yl-10 acétique* (formule 22) $C_{15}H_{17}Cl_2N_2O_2$ P.M. = 292,77

Préparé selon l'exemple 5.1 à partir de 13,6 g (0,054 mole) de chloro-5 coumarine-3 carboxylate d'éthyle, 6,1 g (0,054 mole) de N-méthyl pipéridone-4,8,3 g (0,108 mole) d'acetate d'ammonium et 250 ml d'éthanol. On obtient après recristallisation dans l'éthanol 6,7 g de lactame de l'acide [amino-4a, chloro-9, méthyl-2, hexahydro-1,2,3,4,4a,10a, [10H](benzopyrano)(3,2-C)(pyridine)]-yl-10 acétique. (Rdt = 42,4%) $PF_G$ = 241—243°C $IR_{\nu C=O}$: 1680 $cm^{-1}$.

*Analyse:*

| | C % | H % | Cl % | N % |
|---|---|---|---|---|
| calculée | 61,53 | 5,85 | 12,11 | 9,57 |
| trouvée | 61,36 | 5,76 | 11,98 | 9,61 |

EXEMPLE 20

*Lactame de l'acide [amino-4a, chloro-6, méthyl-2 hexahydro-1,2,3-4,4a,10a, [10H](benzopyrano)-(3,2-C)(pyridine)]-yl-10 acétique* (formule 23) $C_{15}H_{17}ClN_2O_2$ P.M. = 292,77

Préparé selon l'exemple 5.1 à partir de 12 g (0,048 mole) de chloro-8, coumarine-3, carboxylate d'éthyle, 5,5 g (0,048 mole) de N-méthylpipéridone-4, 7,4 g (0,096 mole) d'acétate d'ammonium et 140 ml d'éthanol. On obtient après recristallisation dans l'isopropanol 6 g de lactame de l'acide [amino-4a, chloro-6, méthyl-2, hexahydro-1,2,3,4,4a,10a, [10H](benzopyrano)(3,2-C)pyridine)]-yl-10 acétique (Rdt = 43%) $PF_G$ = 206—208°C $IR_{\nu C=O}$: 1680 $cm^{-1}$.

*Analyse:*

| | C % | H % | Cl % | N % | O % |
|---|---|---|---|---|---|
| calculée | 61,53 | 5,85 | 12,11 | 9,57 | 10,93 |
| trouvée | 61,43 | 5,78 | 12,32 | 9,53 | |

EXEMPLE 21

*Lactame de l'acide [amino-4a, isopropyl-2, hexahydro-1,2,3,4,4a,10a, [10H](benzopyrano)(3,2-C)-(pyridine)]-yl-10a acétique* (formule 24) $C_{17}H_{22}N_2O_2$ P.M. = 286,37

Préparé selon l'exemple 11 à partir de 12,2 g (0,05 mole) du produit de l'exemple 4, 9,35 g (0,055 mole) d'iodure d'isopropyle. On obtient 7,1 g de lactame de l'acide [amino-4a, isopropyl-2, hexahydro-1,2,3,4,4a,10a [10H](benzopyrano)(3,2-C)(pyridine)]-yl-10 acétique (Rdt = 49,5%) $PF_G$ = 214—216°C $IR_{\nu C=O}$: 1680 $cm^{-1}$

*Analyse:*

| | C % | H % | N % |
|---|---|---|---|
| calculée | 71,30 | 7,74 | 9,78 |
| trouvée | 70,92 | 7,70 | 9,78 |

EXEMPLE 22

*Lactame de l'acide [amino-4, éthoxalyl-2, hexahydro-1,2,3,4,4a,10a, [10H](benzopyrano)(3,2-C)-(pyridine)]-yl-10 acétique* (formule 25) $C_{18}H_{20}N_2O_5$ P.M. = 344,37

A une solution de 17,1 g (0,07 mole) du produit de l'exemple 4, 19,6 ml de triéthylamine dans 280 ml de chloroforme, on ajoute en maintenant la température en dessous de 35°C, une solution de 11,4 g (0,084 mole) de chlorure d'éthoxalyle dans 40 ml de chloroforme. On laisse 6 heures à température ambiante. Après un lavage à l'eau, la phase organique est séchée sur $Na_2SO_4$, on évapore ensuite le solvant et le résidu est recristallisé dans le méthanol. On obtient 13,4 g de lactame de l'acide [amino-4a, éthoxalyl-2, hexahydro-1,2,3,4,4a,10a, [10H](benzopyrano)(3,2-C)(pyridine)]-yl-10 acétique (Rdt = 55,5%) $PF_G$ = 210—212°C.

$IR_{\nu C=O}$: 1680 $cm^{-1}$ (lactame)
: 1730 $cm^{-1}$ (ester)

*Analyse:*

| | C % | H % | N % |
|---|---|---|---|
| calculée | 62,78 | 5,85 | 8,13 |
| trouvée | 62,65 | 5,83 | 8,01 |

EXEMPLE 23

*Lactame de l'acide [amino-4a, éthoxycarbonylméthyl-2, hexahydro-1,2,3,4,4a,10a, [10H](benzopyrano)(3,2-C)(pyridine)]-yl-10 acétique* (formule 26) $C_{18}H_{22}N_2O_4$ P.M. = 330,39

Préparé selon l'exemple 11 à partir de 15 g (0,061 mole) du produit de l'exemple 4 et 7,2 ml (0,064 mole) de bromacétate d'éthyle. On obtient après recristallisation dans l'éthanol 12 g de lactame de l'acide [amino-4a, éthoxycarbonylméthyl-2, hexahydro-1,2,3,4,4a,10a, [10H](benzopyrano)(3,2-C)-(pyridine)]-yl-10 acétique (Rdt = 59,5%) $PF_G$ = 198—200°C.

$IR_{\nu C=O}$: 1680 $cm^{-1}$ (lactame)
: 1720 $cm^{-1}$ (ester)

*Analyse:*

| | C % | H % | N % |
|---|---|---|---|
| calculée | 65,44 | 6,71 | 8,48 |
| trouvée | 65,51 | 6,59 | 8,46 |

EXEMPLE 24

*Lactame de l'acide [amino-4a-allyl-2, hexahydro-1,2,3,4,4a,10a, [10H](benzopyrano)(3,2-C)(pyridine)]-yl-10 acétique* (formule 27) $C_{17}H_{20}N_2O_2$ P.M. = 284,36

Préparé selon l'exemple 11 à partir de 15 g (0,061 mole) du produit de l'exemple 4 et 8,1 g (0,067 mole) de bromure d'allyle. On obtient après recristallisation dans l'isopropanol 4,2 g de lactame de l'acide [amino-4a, allyl-2, hexahydro-1,2,3,4,4a,10a, [10H](benzopyrano)(3,2-C)(pyridine)]-yl-10 acétique (Rdt = 25%). $PF_G$ = 206—208°C $IR_{\nu C=O}$: 1680 $cm^{-1}$.

*Analyse:*

| | C % | H % | N % |
|---|---|---|---|
| calculée | 71,85 | 7,09 | 9,85 |
| trouvée | 71,61 | 7,01 | 9,74 |

EXEMPLE 25

*Lactame de l'acide [amino-4a, cinnamoyl-2, hexahydro-1,2,3,4,4a,10a, [10H](benzopyrano)(3,2-C)-(pyridine)]-yl-10 acétique* (formule 28) $C_{23}H_{21}N_2O_3$ P.M. = 374,43

Préparé selon l'exemple 9 à partir de 8,5 g (0,035 mole) du produit de l'exemple 4 et 7 g (0,042 mole) de chlorure de cinnamoyle. On obtient après recristallisation dans l'éthanol 7,4 g de lactame de l'acide [amino-4a, cinnamoyl-2, hexahydro-1,2,3,4,4a,10a, [10H](benzopyrano)(3,2-C)(pyridine)]-yl-10 acétique (Rdt = 56,5%) $PF_G$ = 248—250°C $IR_{\nu C=O}$: 1680 $cm^{-1}$.

*Analyse:*

| | C % | H % | N % |
|---|---|---|---|
| calculée | 73,78 | 5,92 | 7,48 |
| trouvée | 73,72 | 5,98 | 7,40 |

EXEMPLE 26

*Lactame de l'acide [amino-4a, benzyl-2, chloro-8, hexahydro-1,2,3,4,4a,10a, [10H](benzopyrano)-(3,2-C)(pyridine)]-yl-10 acétique* (formule 29) $C_{21}H_{21}ClN_2O_2$ P.M. = 368,88

Préparé selon l'exemple 5.1 à partir de 292 g (1,15 mole) de chloro-6, coumarine-3, carboxylate d'éthyle, 219 g (1,15 mole) de N-benzyl-pipéridone-4, 178 g (2,31 mole) d'acétate d'ammonium et 4 litres d'éthanol. On obtient après recristallisation dans l'éthanol 220,7 g de lactame de l'acide [amino-4a, benzyl-2, chloro-8, hexahydro-1,2,3,4,4a,10a [10H](benzopyrano)(3,2-C)(pyridine)]-yl-10 acétique (Rdt = 52%) $PF_G$ = 137—139°C $IR_{\nu C=O}$: 1680 $cm^{-1}$

*Analyse:*

| | C % | H % | Cl % | N % |
|---|---|---|---|---|
| calculée | 68,38 | 5,74 | 9,61 | 7,59 |
| trouvée | 68,14 | 5,63 | 9,48 | 7,44 |

EXEMPLE 27

*Lactame de l'acide [amino-4a, chloro-8, éthoxycarbonyl-2, hexahydro-1,2,3,4,4a,10a, [10H](benzopyrano)(3,2-C)(pyridine)]-yl-10 acétique* (formule 30) $C_{17}H_{19}ClN_2O_4$ P.M. = 350,81

On porte à reflux une solution de 21,7 g (0,2 mole) de chloroformiate d'éthyle dans 20 ml de benzène. On ajoute alors goutte à goutte une solution de 22 g (0,06 mole) du produit de l'exemple 26 dans 150 ml de benzène. On poursuit le reflux pendant 6 heures. Après refroidissement on lave avec de l'eau puis par HCl 3N et par l'eau. Après séchage sur $Na_2SO_4$ on évapore les solvants. Le résidu est recristallisé dans le méthanol. On obtient 16 g de lactame de l'acide [amino-4a, chloro-8, éthoxycarbonyl-2, hexahydro-1,2,3,4,4a,10a, [10H](benzopyrano)(3,2-C)(pyridine)]-yl-10 acétique (Rdt = 76%) $PF_G$ = 226—228°C. $IR_{\nu C=O}$: 1680 $cm^{-1}$.

*Analyse:*

| | C % | H % | Cl % | N % |
|---|---|---|---|---|
| calculée | 58,20 | 5,46 | 10,10 | 7,99 |
| trouvée | 58,04 | 5,52 | 9,63 | 7,72 |

EXEMPLE 28

*Lactame de l'acide [amino-4a, chloro-8, méthoxycarbonyl-2, hexahydro-1,2,3,4,4a,10a, [10H](benzopyrano)(3,2-C)(pyridine)]-yl-10 acétique* (formule 31) $C_{16}H_{17}ClN_2O_4$ P.M. = 336,77

Préparé selon l'exemple 27 à partir de 22 g (0,06 mole) du produit de l'exemple 26 et 21,7 g (0,2 mole) de chloroformiate de méthyle. On obtient après recristallisation dans l'éthanol 8,3 g de lactame de l'acide [amino-4a, chloro-8, méthoxycarbonyl-2, hexahydro-1,2,3,4,4a,10a, [10H](benzopyrano)-(3,2-C)(pyridine)]-yl-10 acétique (Rdt = 41%) $PF_G$ =165—167°C $IR_{\nu C=O}$: 1680 $cm^{-1}$.

*Analyse:*

| | C % | H % | Cl % | N % |
|---|---|---|---|---|
| calculée | 57,06 | 5,09 | 10,53 | 8,32 |
| trouvée | 57,20 | 5,14 | 10,66 | 8,23 |

EXEMPLE 29

*Lactame de l'acide [amino-4a, chloro-8, butoxycarbonyl-2, hexahydro-1,2,3,4,4a,10a, [10H](benzopyrano)(3,2-C)(pyridine)]-yl-10 acétique* (formule 32) $C_{19}H_{23}ClN_2O_4$ P.M. = 378,85

Préparé selon l'exemple 27 à partir de 17 g (0,046 mole) du produit de l'exemple 26 et 21,2 g (0,155 mole) de chloroformiate de n-butyle. On obtient après recristallisation dans l'acétate d'éthyle 10,2 g de lactame de l'acide [amino-4a, chloro-8, butoxycarbonyl-2, hexahydro-1,2,3,4,4a,10a, [10H](benzopyrano)(3,2-C)(pyridine)]-yl-10 acétique (Rdt = 58,5%) $PF_G$ =144—145°C $IR_{\nu C=O}$: 1680 $cm^{-1}$.

*Analyse:*

| | C % | H % | Cl % | N % |
|---|---|---|---|---|
| calculée | 60,24 | 6,12 | 9,36 | 7,39 |
| trouvée | 60,29 | 6,17 | 9,32 | 7,31 |

EXEMPLE 30

*Lactame de l'acide [amino-4a, chloro-8, (trifluoro-2,2,2 éthoxy) carbonyl-2, hexahydro-1,2,3,4,4a,10a, [10H](benzopyrano)(3,2-C)(pyridine)]-yl-10 acétique* (formule 33) $C_{17}H_{16}ClF_3N_2O_4$ P.M. = 404,78

Préparé selon l'exemple 27 à partir de 5,9 g (0,02 mole) du produit de l'exemple 5,1 et 13,2 g (0,066 mole) de chloroformiate de trifluoroéthyle. On obtient après recristallisation dans l'isopropanol 3 g de lactame de l'acide [amino-4a, chloro-8 (trifluoro-2,2,2 éthoxy) carbonyl-2, hexahydro-1,2,3,4,4a,10a, [10H](benzopyrano)(3,2-C)(pyridine)]-yl-10 acétique (Rdt = 37%) $PF_G$ = 194—196°C
$IR_{\nu C=O}$: 1690 $cm^{-1}$ (lactame)
: 1720 $cm^{-1}$ (carbamate)

*Analyse:*

| | C % | H % | F % | N % |
|---|---|---|---|---|
| calculée | 50,44 | 3,98 | 14,08 | 6,92 |
| trouvée | 50,33 | 3,94 | 14,18 | 6,83 |

EXEMPLE 31

*Lactame de l'acide [amino-4a, chloro-8 (éthyl-2, hexyloxy) carbonyl-2 hexahydro-1,2,3,4,4a,10a [10H](benzopyrano)(3,2-C)(pyridine)]-yl-10 acétique* (formule 34) $C_{23}H_{31}ClN_2O_4$ P.M. = 434,96

Préparé selon l'exemple 27 à partir de 12,2 g (0,0416 mole) du produit de l'exemple 5.1 et 26,7 g (0,139 mole) de chloroformiate d'éthyl-2, hexyle. On obtient après recristallisation dans l'acétone 8,1 g de lactame de l'acide [amino-4a, chloro-8 (éthyl-2 hexyloxy) carbonyl-2, hexahydro-1,2,3,4,4a,10a, [10H](benzopyrano)(3,2-C)(pyridine)]-yl-10 acétique (Rdt = 44,8%) $PF_G$ = 140—142°C.
$IR_{\nu C=O}$: 1690 $cm^{-1}$ (lactame)
: 1710 $cm^{-1}$ (carbamate)

*Analyse:*

| | C % | H % | Cl % | N % |
|---|---|---|---|---|
| calculée | 63,51 | 7,18 | 8,15 | 6,44 |
| trouvée | 63,46 | 7,11 | 8,23 | 6,33 |

EXEMPLE 32

*Lactame de l'acide [amino-4a, chloro-8, cyclohexyloxycarbonyl-2, hexahydro-1,2,3,4,4a,10a, [10H]-(benzopyrano)(3,2-C)(pyridine)]-yl-10 acétique* (formule 35) $C_{21}H_{25}ClN_2O_4$ P.M. = 404,89

Préparé selon l'exemple 27 à partir de 12,2 g (0,0416 mole) du produit de l'exemple 5.1 et 22,6 g (0,139 mole) de chloroformiate de cyclohexyle. On obtient après recristallisation dans l'éthanol 7,2 g de lactame de l'acide [amino-4a, chloro-8, cyclohexyloxycarbonyl-2, hexahydro-1,2,3,4,4a,10a, [10H](benzopyrano)(3,2-C)(pyridine)]-yl-10 acétique (Rdt = 42,7%) $PF_G$ = 222—224°C $IR_{\nu C=O}$: 1690 $cm^{-1}$.

*Analyse:*

| | C % | H % | Cl % | N % |
|---|---|---|---|---|
| calculée | 62,29 | 6,22 | 8,75 | 6,92 |
| trouvée | 62,43 | 6,22 | 8,80 | 6,87 |

EXEMPLE 33

*Lactame de l'acide [amino-4a, chloro-8, benzyloxycarbonyl-2, hexahydro-1,2,3,4,4a,10a, [10H](benzopyrano)(3,2-C)(pyridine)]-yl-10 acétique* (formule 36) $C_{22}H_{21}ClN_2O_4$ P.M. = 412,86

Préparé selon l'exemple 27 à partir de 36,9 g (0,1 mole) du produit de l'exemple 26 et 56,5 g (0,33 mole) de chloroformiate de benzyle. On obtient après recristallisation dans l'acétate d'éthyle 19 g de lactame de l'acide [amino-4a, chloro-8, benzyloxycarbonyl-2, hexahydro-1,2,3,4,4a,10a, [10H](benzopyrano)(3,2-C)(pyridine)]-yl-10 acétique (Rdt = 46%) $PF_G$ = 228—230°C.

$IR_{\nu C=O}$: 1680 $cm^{-1}$ (lactame)
: 1720 $cm^{-1}$ (carbamate)

*Analyse:*

| | C % | H % | Cl % | N % |
|---|---|---|---|---|
| calculée | 64,02 | 5,12 | 8,58 | 6,78 |
| trouvée | 63,85 | 5,28 | 8,31 | 6,62 |

EXEMPLE 34

*Lactame de l'acide [amino-4a, chloro-8, hexahydro-1,2,3,4,4a,10a, [10H](benzopyrano)(3,2-C)(pyridine)]-yl-10 acétique* (formule 37) $C_{14}H_{15}ClN_2O_2$ P.M. = 278,73

On solubilise 27,8 g (0,06 mole) du produit de l'exemple 33 dans 400 $cm^3$ de chloroforme et l'on fait barboter de l'acide bromhydrique (obtenu par chauffage à 50°C d'une solution aqueuse à 62%) pendant 30 minutes, à température ambiante. Un précipité se forme. On le filtre, on le solubilise dans 3 l d'eau et on basifie avec NaOH 10%. Par refroidissement on obtient un précipité. On l'essore, on le sèche, on le recristallise dans le méthanol. On obtient 10 g de lactame de l'acide [amino-4a, chloro-8, hexahydro-1,2,3,4,4a,10a, [10H](benzopyrano)(3,2-C)(pyridine)]-yl-10 acétique (Rdt = 59,8%) $PF_G$ = 252—254°C $IR_{\nu C=O}$: 1680 $cm^{-1}$.

*Analyse:*

| | C % | H % | Cl % | N % |
|---|---|---|---|---|
| calculée | 60,33 | 5,42 | 12,72 | 10,05 |
| trouvée | 60,32 | 5,30 | 12,57 | 10,08 |

EXEMPLE 35

*Lactame de l'acide [amino-4a, chloro-8, phényl-2, hexahydro-1,2,3,4,4a,10a, [10H](benzopyrano)-(3,2-C)(pyridine)]-yl-10 acétique* (formule 38) $C_{20}H_{19}ClN_2O_2$ P.M. = 354,83

Préparé selon l'exemple 5.1 à partir de 16,9 g (0,067 mole) de chloro-6, coumarine-3 carboxylate d'éthyle, 11,7 g (0,067 mole) de N-phényl pipéridone-4, 10,5 g (0,134 mole) d'acétate d'ammonium et 480 ml d'éthanol. Par recristallisation dans le méthanol on obtient 9,5 g de lactame de l'acide [amino-4a, chlorro-8, phényl-2, hexahydro-1,2,3,4,4a,10a, [10H](benzopyrano)(3,2-C)(pyridine)]-yl-10 acétique (Rdt = 40%) $PF_G$ =220—222°C $IR_{\nu C=O}$: 1680 $cm^{-1}$.

*Analyse:*

| | C % | H % | Cl % | N % |
|---|---|---|---|---|
| calculée | 67,70 | 5,40 | 9,99 | 7,89 |
| trouvée | 67,83 | 5,48 | 9,86 | 7,86 |

EXEMPLE 36

*Lactame de l'acide [chloro-8, méthyl-2, méthylamino-4a, hexahydro-1,2,3,4,4a,10a, [10H](benzopyrano)(3,2-C)(pyridine)]-yl-10 acétique* (formule 39) $C_{16}H_{19}ClN_2O_2$ P.M. = 306,79

On place dans un autoclave 33 g (0,125 mole) de chloro-6 coumarine-3 carboxylate d'éthyle, 14,3 g de N-méthyl, pipéridone-4, 24 g de méthylamine (0,25 mole) en solution à 33% dans l'éthanol et 550 ml d'éthanol. On porte 7 heures à 70—80°C. Après refroidissement on traite comme dans l'exemple 5.1. On obtient après recristallisation dans l'éthanol 13 g de lactame de l'acide [chloro-8, méthyl-2 méthylamino-4a, hexahydro-1,2,3,4,4a,10a, [10H](benzopyrano)(3,2-C)(pyridine)]-yl-10 acétique (Rdt = 34%) $PF_G$ = 158—160°C $IR_{\nu C=O}$: 1680 $cm^{-1}$.

*Analyse:*

| | C % | H % | Cl % | N % |
|---|---|---|---|---|
| calculée | 62,64 | 6,24 | 11,56 | 9,13 |
| trouvée | 62,70 | 6,36 | 11,48 | 9,20 |

EXEMPLE 37

*Lactame de l'acide [méthyl-2, méthylamino-4a, hexahydro-1,2,3,4,4a,10a, [10H](benzopyrano)-(3,2-C)(pyridine)]-yl-10 acétique* (formule 40) $C_{16}H_{20}N_2O_2$ P.M. = 272,34

Préparé selon l'exemple 36 à partir de 27,3 g (0,125 mole) de coumarine-3 carboxylate d'éthyle, 14,3 g de N-méthyl, pipéridone-4, 24 g (0,25 mole) de méthylamine en solution à 33% dans l'éthanol

et 500 ml d'éthanol. On obtient une base brute qu'il n'a pas été possible de recristalliser: 25 g

*citrate* $C_{22}H_{28}N_2O_9$ P.M. = 464,48

On solubilise les 25 g de produit brut dans 120 ml d'acétone et on ajoute sous refroidissement une solution de 19,2 g (0,1 mole) d'acide citrique dans 200 ml d'acétone. On essore le produit obtenu et on le recristallise dans l'éthanol. On obtient 15 g de citrate du lactame de l'acide [méthyl-2, méthylamino-4a, hexahydro-1,2,3,4,4a,10a, [10H](benzopyrano)(3,2-C)(pyridine)]-yl-10 acétique (Rdt = 24% $PF_G$ = 180—182°C $IR_{\nu C=O}$: 1690 $cm^{-1}$.

*Analyse:*

| | C % | H % | N % |
|---|---|---|---|
| calculée | 56,89 | 6,07 | 6,03 |
| trouvée | 56,69 | 5,97 | 5,92 |

## EXEMPLE 38

*Lactame de l'acide [benzylamino-4a, méthyl-2, chloro-8, hexahydro-1,2,3,4,4a,10a [10H](benzopyrano)(3,2-C)(pyridine)]-yl-10 acétique* (formule 41) $C_{22}H_{23}ClN_2O_2$ P.M. = 382,88

Préparé selon l'exemple 5.1 à partir de 25,2 g (0,1 mole) de chloro-6, coumarine-3 carboxylate d'éthyle, 11,5 g (0,1 mole) de N'méthyl pipéridone-4, 21,4 g (0,2 mole) de benzylamine et 500 ml d'éthanol. Après recristallisation dans le diisopropyléther on obtient 9,5 g de lactame de l'acide [benzylamino-4a, chloro-8, méthyl-2, hexahydro-1,2,3,4,4a,10a, [10H](benzopyrano)(3,2-C)-(pyridine)]-yl-10 acétique (Rdt = 25%) $PF_G$ = 115—117°C $IR_{\nu C=O}$: 1690 $cm^{-1}$.

*Analyse:*

| | C % | H % | Cl % | N % |
|---|---|---|---|---|
| calculée | 69,01 | 6,05 | 9,26 | 7,32 |
| trouvée | 69,14 | 6,15 | 9,07 | 7,17 |

## EXEMPLE 39

*Lactame de l'acide [amino-4a, chloro-8, (trichloro-2,2,2 éthoxy) carbonyl-2, hexahydro-1,2,3,4,4a,10a, [10H](benzopyrano)(3,2-C)(pyridine)]-yl-10 acétique* (formule 42) $C_{17}H_{16}Cl_4N_2O_4$ P.M. = 454,14

Préparé selon l'exemple 27 à partir de 12,2 g (0,0416 mole) du produit de l'exemple 5.1, et 29,5 g (0,139 mole) de chloroformiate de trichloroéthyle. Par recristallisation dans l'acétate d'éthyle on obtient, 8,4 g de lactame de l'acide [amino-4a, chloro-8, (trichloro-2,2,2, éthoxy) carbonyl-2, hexahydro-1,2,3,4,4a,10a, [10H](benzopyrano)(3,2-C)(pyridine)]-yl-10 acétique (Rdt = 44,5%) $PF_G$ = 223—225°C.

$IR_{\nu C=O}$: 1695 $cm^{-1}$ (lactame)

: 1720 $cm^{-1}$ (carbamate)

*Analyse:*

| | C % | H % | Cl % | N % |
|---|---|---|---|---|
| calculée | 44,96 | 3,55 | 31,23 | 6,16 |
| trouvée | 45,12 | 3,38 | 31,46 | 5,92 |

## EXEMPLE 40

*Lactame de l'acide [amino-4a, benzoyl-2, hexahydro-1,2,3,4,4a,10a, [10H](benzopyrano)(3,2-C)-(pyridine)]-yl-10 acétique* (formule 43) $C_{21}H_{20}N_2O_3$ P.M. = 348,40

Préparé selon l'exemple 9 à partir de 8,5 g (0,035 mole) du produit de l'exemple 4 et 5,9 g (0,042 mole) de chlorure de benzoyle. Par recristallisation dans l'éthanol on obtient 8,2 g de lactame de l'acide [amino-4a, benzoyl-2, hexahydro-1,2,3,4,4a,10a, [10H](benzopyrano)(3,2-C)(pyridine)]-yl-10 acétique (Rdt = 67%) $PF_G$ = 253—255°C $IR_{\nu C=O}$: 1680 $cm^{-1}$.

*Analyse:*

| | C % | H % | N % |
|---|---|---|---|
| calculée | 72,40 | 5,78 | 8,04 |
| trouvée | 72,29 | 5,51 | 7,91 |

## EXEMPLE 41

*Lactame de l'acide [amino-4a, pivaloyl-2, hexahydro-1,2,3,4,4a,10a, [10H](benzopyrano)(3,2-C)(pyridine)]-yl-10 acétique* (formule 44) $C_{19}H_{24}N_2O_3$ P.M. = 328,41

Préparé selon l'exemple 9 à partir de 8,5 g (0,035 mole) du produit de l'exemple 4 et 5,1 g (0,042 mole) de chlorure de pivaloyle. Après recristallisation dans l'isopropanol on obtient 6,6 g de lactame de l'acide [amino-4a, pivaloyl-2, hexahydro-1,2,3,4,4a,10a, [10H](benzopyrano)(3,2-C)(pyridine)]-yl-10 acétique (Rdt = 57,5%) $PF_G$ = 253—255°C $IR_{\nu C=O}$: 1690 $cm^{-1}$

*Analyse:*

| | C % | H % | N % |
|---|---|---|---|
| calculée | 69,49 | 7,36 | 8,53 |
| trouvée | 69,62 | 7,51 | 8,74 |

EXEMPLE 42

*Lactame de l'acide [amino-4a, acétyl-2, chloro-8, hexahydro-1,2,3,4,4a,10a, [10H](benzopyrano)-(3,2-C)(pyridine)]-yl-10 acétique* (formule 45) $C_{16}H_{17}ClN_2O_3$ P.M. = 320,77

Préparé selon l'exemple à partir de 4,5 g (0,016 mole) du produit de l'exemple 34 et 2 g (0,026 mole) de chlorure d'acétyle. On obtient après recristallisation dans le mélange acétate d'éthyle-méthanol 27 g de lactame de l'acide [amino-4a, acétyl-2, chloro-8, hexahydro-1,2,3,4,4a,10a, [10H](benzopyrano)(3,2-C)(pyridine)]-yl-10 acétique (Rdt = 52,6%) $PF_G$ = 255—257°C
$IR_{\nu C=O}$: 1670 $cm^{-1}$
: 1690 $cm^{-1}$

*Analyse:*

| | C % | H % | Cl % | N % |
|---|---|---|---|---|
| calculée | 59,91 | 5,34 | 11,05 | 8,73 |
| trouvée | 60,12 | 5,51 | 11,32 | 9,01 |

EXEMPLE 43

*Lactame de l'acide [amino-4a, chloro-8, isopropyl-2, hexahydro-1,2,3,4,4a,10a, [10H](benzopyrano)-(3,2-C)(pyridine)]-yl-10 acétique* (formule 46) $C_{17}H_{21}ClN_2O_2$ P.M. = 320,82

Préparé selon l'exemple 11 à partir de 6,2 g (0,022 mole) du produit de l'exemple 34 et 3,1 g de bromo-2 propane. On obtient après recristallisation dans l'éthanol 2 g de lactame de l'acide [amino-4a, chloro-8, isopropyl-2, hexahydro-1,2,3,4,4a,10a, [10H](benzopyrano)(3,2-C)(pyridine)]-yl-10-10 acétique (Rdt = 28%) $PF_G$ = 186—188°C $IR_{\nu C=O}$: 1680 $cm^{-1}$.

*Analyse:*

| | C % | H % | Cl % | N % |
|---|---|---|---|---|
| calculée | 63,65 | 6,60 | 11,05 | 8,73 |
| trouvée | 63,44 | 6,39 | 10,88 | 8,55 |

EXEMPLE 44

*Lactame de l'acide [amino-4a, chloro-8, (méthyl-2, propényl-3)2, hexahydro-1,2,3,4,4a,10a, [10H]-(benzopyrano)(3,2-C)(pyridine)]-yl-10 acétique* (formule 47) $C_{18}H_{21}ClN_2O_2$ P.M. = 332,83

Préparé selon l'exemple 11 à partir de 6,2 g (0,022 mole) du produit de l'exemple 34 et 2,6 ml (0,025 mole) de chloro-3, méthyl-2 propène. Par recristallisation dans l'acétate d'éthyle on obtient 2,3 g de lactame de l'acide [amino-4a, chloro-8, (méthyl-2, propényl-3) 2, hexahydro-1,2,3,4,4a,10a, [10H](benzopyrano)(3,2-C)(pyridine)]-yl-10 acétique (Rdt: 31,5%) $PF_G$ = 202—204°C $IR_{\nu C=O}$: 1680 $cm^{-1}$

*Analyse:*

| | C % | H % | Cl % | N % |
|---|---|---|---|---|
| calculée | 64,95 | 6,36 | 10,65 | 8,42 |
| trouvée | 65,12 | 6,47 | 10,72 | 8,51 |

**Revendications**

1. Hexahydro benzopyrano [3,2-C] pyridines substituées caractérisées par la formule

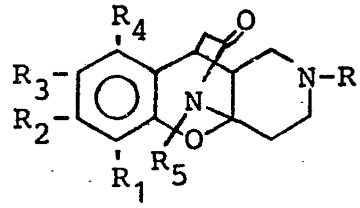

dans laquelle R est l'hydrogène ou un radical alcoyle inférieur saturé ou insaturé, linéaire ou ramifié, aralkyle, acyle, dialkylaminoalkyle, alkylcarbonyle, alcoxycarbonyle, halogénoalcoxycarbonyle, aryle, benzyloxycarbonyle, éthoxalyle et éthoxycarbonylméthyle; $R_1$ est l'hydrogène, un halogène ou un radical alcoxy inférieur; $R_2$ est l'hydrogène ou un halogène; $R_3$ est l'hydrogène, un halogène, un radical alcoyle inférieur, alcoxy, nitro, amino ou forme le naphtalène avec $R_4$ et le cycle benzénique; $R_4$ est l'hydrogène, un halogène ou forme le naphtalène avec $R_3$ et le cycle benzénique; $R_5$ est l'hydrogène, un radical alcoyle inférieur ou arylalkyle.

2. Hexahydro benzopyrano [3,2-C] pyridines selon la revendication 1, caractérisé en ce qu'elles sont sous forme de leurs sels avec des acides minéraux et organiques acceptables en thérapeutique humaine.

3. Lactame de l'acide [amino-4a chloro-8 méthyl-2 hexahydro-1,2,3,4,4a,10a [10H](benzopyrano)(3,2-C)(pyridine)]-yl-10 acétique selon la revendication 2.

4. Lactame de l'acide [amino-4a bromo-8 méthyl-2 hexahydro-1,2,3,4,4a,10a [10H](benzopyrano)(3,2-C)(pyridine)]-yl-10 acétique selon la revendication 2.

5. Lactame de l'acide [amino-4a fluoro-8 méthyl-2 hexahydro-1,2,3,4,4a,10a [10H](benzopyrano)(3,2-C)(pyridine)]-yl-10 acétique selon la revendication 2.

6. Lactame de l'acide [amino-4a nitro-8 méthyl-2 hexahydro-1,2,3,4,4a,10a [10H](benzopyrano)(3,2-C)(pyridine)]-yl-10 acétique selon la revendication 2.

7. Lactame de l'acide [amino-4a méthoxy-8 méthyl-2 hexahydro-1,2,3,4,4a,10a [10H](benzopyrano)(3,2-C)(pyridine)]-yl-10 acétique selon la revendication 2.

8. Lactame de l'acide [amino-4a chloro-8 éthoxy-carbonyl-2 hexahydro-1,2,3,4,4a,10a [10H]-(benzopyrano)(3,2-C)(pyridine)]-yl-10 acétique selon la revendication 2.

9. Lactame de l'acide [amino-4a chloro-8 isopropyl-2 hexahydro-1,2,3,4,4a,10a [10H](benzopyrano)(3,2-C)(pyridine)]-yl-10 acétique selon la revendication 2.

10. Lactame de l'acide [chloro-8-méthyl-2 méthylamino-4a, hexahydro-1,2,3,4,4a,10a [10H]-(benzopyrano)(3,2-c)(pyridine)]-yl-10 acétique selon la revendication 2.

11. Procédé de préparation des hexahydro benzopyrano [3,2-C] pyridines selon la revendication 1, caractérisé en ce qu'on additionne une pipéridone-4N-substituée de formule

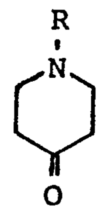

dans laquelle R a la même signification que précédemment sur une coumarine carboxylate d'éthyl-3 de formule

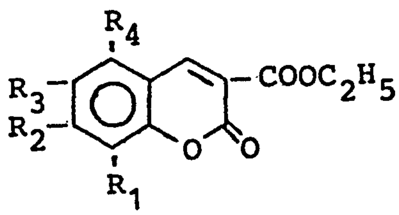

dans laquelle $R_1$, $R_2$, $R_3$, $R_4$ ont lers mêmes significations que précédemment, ouvre par l'acétate d'ammonium l'adduct résultant ou par une amine primaire $R_5$—$NH_2$ dans laquelle $R_5$ a la même signification que précédemment, puis effectue une cyclisation déshydratante par l'acide chlorhydrique.

12. Médicament caractérisé en ce qu'il contient comme principe actif une hexahydro benzopyrano [3,2-C] pyridine selon la revendication 1 ou 2.

13. Médicament selon la revendication 12, caractérisé en ce qu'il contient comme principe actif le lactame de l'acide [amino-4a chloro-8 méthyl-2 hexahydro-1,2,3,4,4a,10a, [10H](benzopyrano)(3,2-C)(pyridine)]-yl-10 acétique.

14. Médicament selon la revendication 12, caractérise en ce qu'il contient comme principe actif le lactame de l'acide [amino-4a bromo-8 méthyl-2 hexahydro-1,2,3,4,4a,10a [10H](benzopyrano)(3,2-C)-(pyridine)]-yl-10 acétique.

15. Médicament selon la revendication 12, caractérisé en ce qu'il contient comme principe actif le lactame de l'acide [amino-4a fluoro-8 méthyl-2 hexahydro-1,2,3,4,4a,10a, [10H](benzopyrano)(3,2-C)-(pyridine)]-yl-10 acétique.

16. Médicament selon la revendication 12, caractérisé en ce qu'il contient comme principe actif le lactame de l'acide [amino-4a nitro-8 méthyl-2 hexahydro-1,2,3,4,4a,10a, [10H](benzopyrano)(3,2-C)-(pyridine)]-yl-10 acétique.

17. Médicament selon la revendication 12, caractérisé en ce qu'il contient comme principe actif le lactame de l'acide [amino-4a méthoxy-8 méthyl-2 hexahydro-1,2,3,4,4a,10a, [10H]benzopyrano(3,2-C)(pyridine)]-yl-10 acétique.

18. Médicament selon la revendication 12, caractérisé en ce qu'il contient comme principe actif le lactame de l'acide [amino-4a chloro-8 éthoxycarbonyl-2 hexahydro-1,2,3,4,4a,10a, [10H]benzopyrano(3,2-C)(pyridine)]-yl-10 acétique.

19. Médicament selon la revendication 12, caractérisé en ce qu'il contient comme principe actif le

lactame de l'acide [chloro-8 méthyl-2 méthylamino-4a, hexahydro-1,2,3,4,4a,10a [10H](benzopyrano)(3,2-C)(pyridine)]yl-10 acétique.

## Patentansprüche

1. Substituierte Hexahydrobenzopyrano[3,2-C]pyridine der Formel

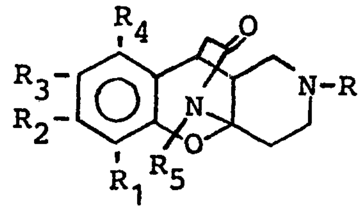

worin R ein Wasserstoffatom oder ein niedermolekularer gesättigter oder ungesättigter, geradkettiger oder verzweigtkettiger Alkylrest, ein Aralkylrest, ein Acylrest, ein Dialkylaminoalkylrest, ein Alkylcarbonylrest, ein Alkoxycarbonylrest, ein Halogenalkoxycarbonylrest, ein Arylrest, ein Benzyloxycarbonylrest, ein Äthoxyalkylrest oder Äthoxycarbonylmethylrest ist, $R_1$ ein Wasserstoffatom, ein Halogenatom oder ein niedermolekularer Alkoxyrest ist, $R_2$ ein Wasserstoffatom oder Halogenatom ist, $R_3$ ein Wasserstoffatom, ein Halogenatom, ein niedermolekularer Alkylrest, ein Alkoxyrest, ein Nitrorest oder Aminorest ist oder mit $R_4$ und dem Benzolring den Naphthalinrest bildet, $R_4$ ein Wasserstoffatom oder Halogenatom ist oder mit $R_3$ und dem Benzolring einen Naphthalinrest bildet und $R_5$ ein Wasserstoffatom, ein niedermolekularer Alkylrest oder ein Arylalkylrest ist.

2. Hexahydrobenzopyrano[3,2-C]pyridine nach Anspruch 1, dadurch gekennzeichnet, daß sie in der Form ihrer Salze mit den Säureresten von für den Menschen therapeutisch verträglichen Mineralsäuren oder organischen Säuren vorliegen.

3. Lactam der (4a-Amino-8-chlor-2-methyl-1,2,3,4,4a,10a-hexahydro-[10H]-benzopyrano-[3,2-C]pyridin)-10-yl-essigsäure nach Anspruch 2.

4. Lactam der (4a-Amino-8-brom-2-methyl-1,2,3,4,4a,10a-hexahydro-[10H]-benzopyrano-[3,2-C]pyridin)-10-yl-essigsäure nach Anspruch 2.

5. Lactam der (4a-Amino-8-fluor-2-methyl-1,2,3,4,4a,10a-hexahydro-[10H]-benzopyrano-[3,2-C]pyridin)-10-yl-essigsäure nach Anspruch 2.

6. Lactam der (4a-Amino-8-nitro-2-methyl-1,2,3,4,4a,10a-hexahydro-[10H]-benzopyrano-[3,2-C]pyridin)-10-yl-essigsäure nach Anspruch 2.

7. Lactam der (4a-Amino-8-methoxy-2-methyl-1,2,3,4,4a,10a-hexahydro-[10H]-benzopyrano-[3,2-C]pyridin)-10-yl-essigsäure nach Anspruch 2.

8. Lactam der (4a - Amino - 8 - chlor - 2 - äthoxycarbonyl - 1,2,3,4,4a,10a - hexahydro - [10H]-benzopyrano[3,2-C]pyridin) - 10 - yl - essigsäure nach Anspruch 2.

9. Lactam der (4a-Amino-8-chlor-2-isopropyl-1,2,3,4,4a,10a-hexahydro-[10H]-benzopyrano-[3,2-C]pyridin)-10-yl-essigsäure nach Anspruch 2.

10. Lactam der (8-Chlor-2-methyl-4a-methylamino-1,2,3,4,4a,10a-hexahydro-[10H]-benzopyrano[3,2-C]pyridin)-10-yl-essigsäure nach Anspruch 2.

11. Verfahren zur Herstellung von Hexahydrobenzopyrano[3,2-C]pyridinen nach Anspruch 1, dadurch gekennzeichnet, daß man ein substituiertes 4N-Piperidon der Formel

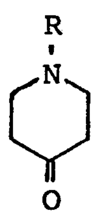

worin R die obige Bedeutung hat, an einen Cumarin-3-carbonsäureäthylester der Formel

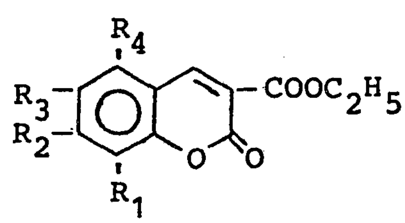

worin $R_1$, $R_2$, $R_3$ und $R_4$ die obige Bedeutung haben, addiert, das resultierende Addukt mit Ammoniumacetat oder einem primären Amin $R_5$—$NH_2$, worin $R_5$ die obige Bedeutung hat, ringöffnet und sodann mit Chlorwasserstoffsäure eine wasserabspaltende Zyklisierung durchführt.

12. Arzneimittel, dadurch gekennzeichnet, daß es als Wirkstoff ein Hexahydrobenzopyrano[3,2-C]pyridin nach Anspruch 1 oder 2 enthält.

13. Arzneimittel nach Anspruch 12, dadurch gekennzeichnet, daß es als Wirkstoff das Lactam der (4a-Amino-8-chlor-2-methyl-1,2,3,4,4a,10a-hexahydro-[10H]-benzopyrano[3,2-C]pyridin)-10-yl-essigsäure enthält.

14. Arzneimittel nach Anspruch 12, dadurch gekennzeichnet, daß es als Wirkstoff das Lactam der (4a-Amino-8-brom-2-methyl-1,2,3,4,4a,10a-hexahydro-[10H]-benzopyrano[3,2-C]pyridin)-10-yl-essigsäure enthält.

15. Arzneimittel nach Anspruch 12, dadurch gekennzeichnet, daß es als Wirkstoff das Lactam der (4a-Amino-8-fluor-2-methyl-1,2,3,4,4a,10a-hexahydro-[10H]-benzopyrano[3,2-C]pyridin)-10-yl-essigsäure enthält.

16. Arzneimittel nach Anspruch 12, dadurch gekennzeichnet, daß es als Wirkstoff das Lactam der (4a-Amino-8-nitro-2-methyl-1,2,3,4,4a,10a-hexahydro-[10H]-benzopyrano[3,2-C]pyridin)-10-yl-essigsäure enthält.

17. Arzneimittel nach Anspruch 12, dadurch gekennzeichnet, daß es als Wirkstoff das Lactam der (4a - Amino - 8 - methoxy- 2 - methyl - 1,2,3,4,4a,10a - hexahydro - [10H] - benzopyrano[3,2-C]-pyridin) - 10 - yl - essigsäure enthält.

18. Arzneimittel nach Anspruch 12, dadurch gekennzeichnet, daß es als Wirkstoff das Lactam der (4a-Amino-8-chlor-2-äthoxycarbonyl-1,2,3,4,4a,10a-hexahydro-[10H]-benzopyrano[3,2-C]pyridin)-10-yl-essigsäure enthält.

19. Arzneimittel nach Anspruch 12, dadurch gekennzeichnet, daß es als Wirkstoff das Lactam der (8-Chlor-2-methyl-4a-methylamino-1,2,3,4,4a,10a-hexahydro-[10H]-benzopyrano[3,2-C]pyridin)-10-yl-essigsäure enthält.

**Claims**

1. Substituted hexahydro benzopyrano[3,2-C]pyridines having the formula

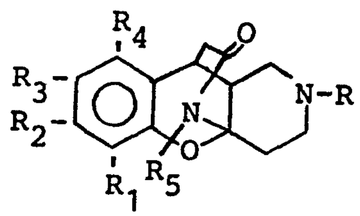

wherein R is hydrogen or a saturated or unsaturated, straight chain or branched lower alkyl radical, an aralkyl radical, an acyl radical, a dialkyl amino alkyl radical, an alkyl carbonyl radical, an alkoxy carbonyl radical, a haloalkoxy carbonyl radical, an aryl radical, a benzyloxy carbonyl radical, an ethoxy alkyl radical or ethoxy carbonylmethyl radical, $R_1$ is hydrogen, halogen or a lower alkoxy radical, $R_2$ is hydrogen or halogen, $R_3$ is hydrogen, halogen, a lower alkyl radical, an alkoxy radical, a nitro radical or amino radical or forms together with $R_4$ and the benzene ring a naphthalene radical, $R_4$ is hydrogen or halogen or forms together with $R_3$ and the benzene ring a naphthalene radical, and $R_5$ is hydrogen, a lower alkyl radical or an arylalkyl radical.

2. Hexahydro benzopyrano[3,2-C]pyridines according to claim 1, characterized in that they exist in the form of the salts thereof with acid radical of mineral acids or organic acids therapeutically acceptable for human beings.

3. Lactam of the (4a-amino-8-chloro-2-methyl-1,2,3,4,4a,10a-hexahydro-[10H]-benzopyrano-[3,2-C]pyridine)-10-yl-acetic acid according to claim 2.

4. Lactam of the (4a-amino-8-bromo-2-methyl-1,2,3,4,4a,10a-hexahydro-[10H]-benzopyrano-[3,2-C]pyridine)-10-yl-acetic acid according to claim 2.

5. Lactam of the (4a-amino-8-fluoro-2-methyl-1,2,3,4,4a,10a-hexahydro-[10H]-benzopyrano-[3,2-C]pyridine)-10-yl-acetic acid according to claim 2.

6. Lactam of the (4a-amino-8-nitro-2-methyl-1,2,3,4,4a,10a-hexahydro-[10H]-benzopyrano-[3,2-C]pyridine)-10-yl-acetic acid according to claim 2.

7. Lactam of the (4a-amino-8-methoxy-2-methyl-1,2,3,4,4a,10a-hexahydro-[10H]-benzopyrano-[3,2-C]pyridine)-10-yl-acetic acid according to claim 2.

8. Lactam of the (4a-amino-8-chloro-2-ethoxycarbonyl-1,2,3,4,4a,10a-hexahydro-[10H]-benzopyrano[3,2-C]pyridine)-10-yl-acetic acid according to claim 2.

9. Lactam of the (4a-amino-8-chloro isopropyl-1,2,3,4,4a,10a-hexahydro-[10H]-benzopyrano-[3,2-C]pyridine)-10-yl-acetic acid according to claim 2.

10. Lactam of the (8-chloro-2-methyl-4a-methylamino-1,2,3,4,4a,10a-hexahydro-[10H]-benzopyrano[3,2-C]pyridine)-10-yl-acetic acid according to claim 2.

11. Process for the production of hexahydro benzopyrano[3,2-C]pyridines according to claim 1, characterized in that a substituted 4N-piperidone having the formula

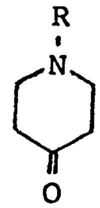

wherein R has the meaning as identified above is added to a coumarin-3-carboxylic acid ethyl ester having the formula

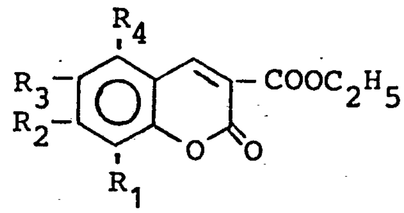

wherein $R_1$, $R_2$, $R_3$, and $R_4$ have the meaning as identified above, the resulting adduct is opened by means of ammonium acetate or a primary amine having the formula $R_5$—$NH_2$, wherein $R_5$ has the meaning identified above, and then a dehydrating cyclisation by means of hydrogen chloride is carried out.

12. Pharmaceutical preparation, characterized in that it contains as active ingredient a hexahydro benzopyrano[3,2-C]pyridine according to claim 1 or 2.

13. Pharmaceutical preparation according to claim 12, characterized in that it contains as active ingredient the lactam of the (4a-amino-8-chloro-2-methyl-1,2,3,4,4a,10a-hexahydro-[10H]-benzopyrano[3,2-C]pyridine)-10-yl-acetic acid.

14. Pharmaceutical preparation according to claim 12, characterized in that it contains as active ingredient the lactam of the (4a-amino-8-bromo-2-methyl-1,2,3,4,4a,10a-hexahydro-[10H]-benzopyrano[3,2-C]pyridine)-10-yl-acetic acid.

15. Pharmaceutical preparation according to claim 12, characterized in that it contains as active ingredient the lactam of the (4a-amino-8-fluoro-2-methyl-1,2,3,4,4a,10a-hexahydro-[10H]-benzopyrano[3,2-C]pyridine)-10-yl-acetic acid.

16. Pharmaceutical preparation according to claim 12, characterized in that it contains as active ingredient the lactam of the (4a-amino-8-nitro-2-methyl-1,2,3,4,4a,10a-hexahydro-[10H]-benzopyrano[3,2-C]pyridine)-10-yl-acetic acid.

17. Pharmaceutical preparation according to claim 12, characterized in that it contains as active ingredient the lactam of the (4a-amino-8-methoxy-2-methyl-1,2,3,4,4a,10a-hexahydro-[10H]-benzopyrano[3,2-C]pyridine)-10-yl-acetic acid.

18. Pharmaceutical preparation according to claim 12, characterized in that it contains as active ingredient the lactam of the (4a-amino-8-chloro-2-ethoxy carbonyl-1,2,3,4,4a,10a-hexahydro-[10H]-benzopyrano[3,2-C]pyridine)-10-yl-acetic acid.

19. Pharmaceutical preparation according to claim 12, characterized in that it contains as active ingredient the lactam of the (8-chloro-2-methyl-4a-methylamino-1,2,3,4,4a,10a-hexahydro-[10H]-benzopyrano[3,2-C]pyridine)-10-yl-acetic acid.

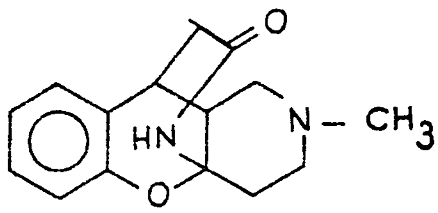

Formule 1

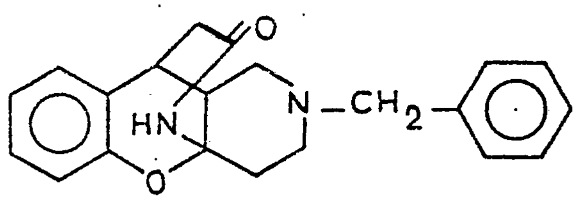

Formule 2

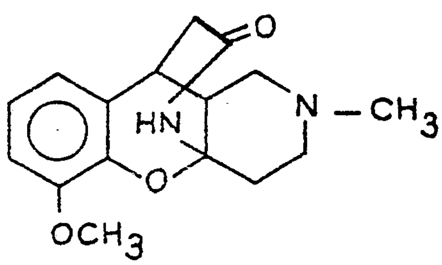

Formule 3

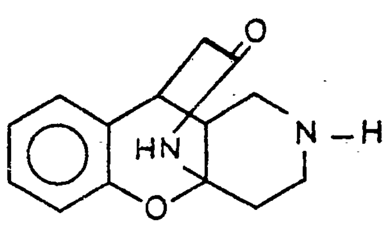

Formule 4

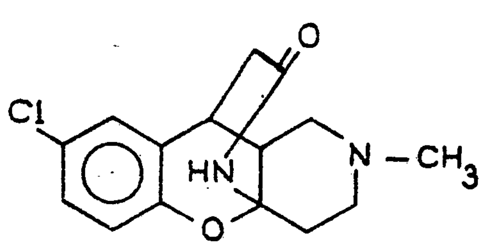

Formule 5

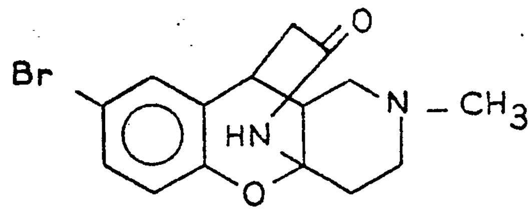

Formule 6

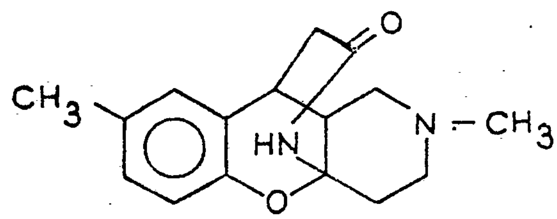

Formule 7

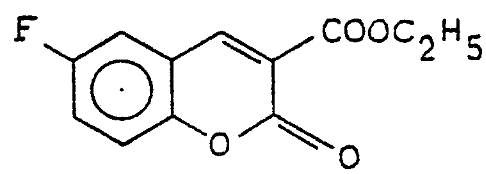

Formule 8

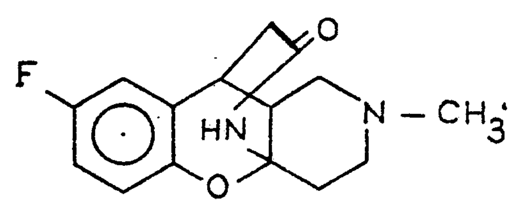

Formule 9

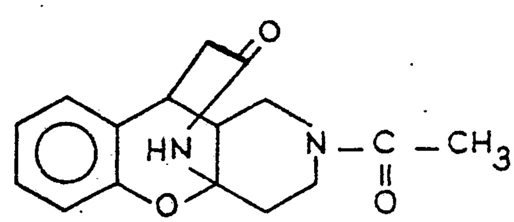

Formule 10

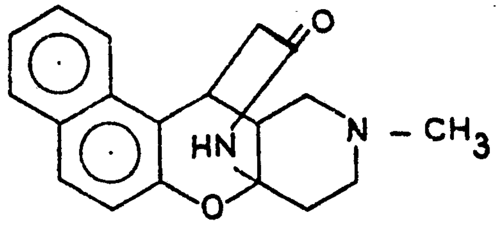

Formule 11

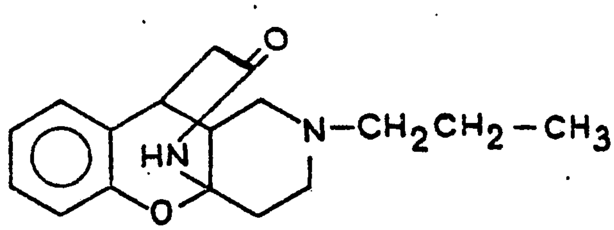

Formule 12

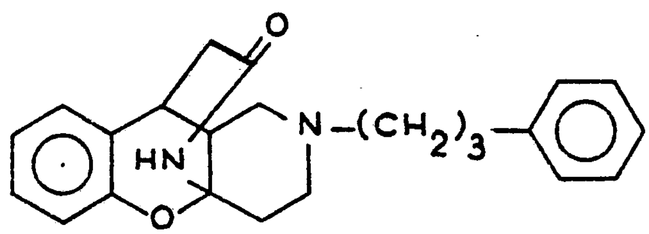

Formule 13

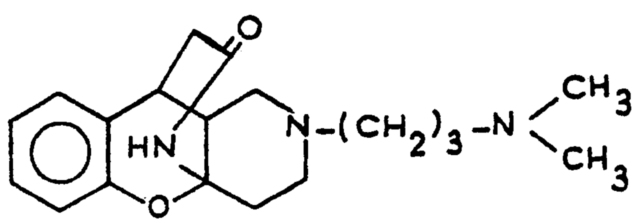

Formule 14

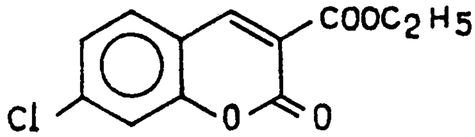

Formule 15

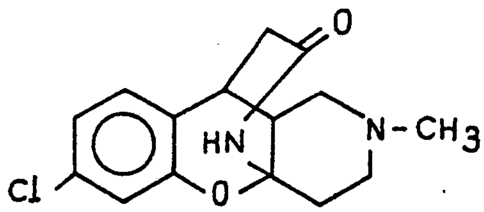

Formule 16

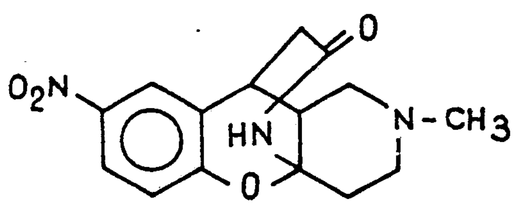

Formule 17

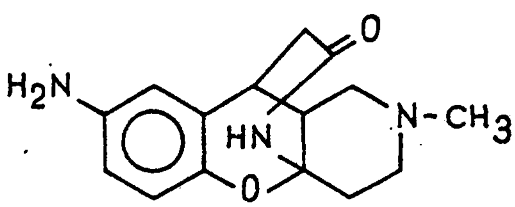

Formule 18

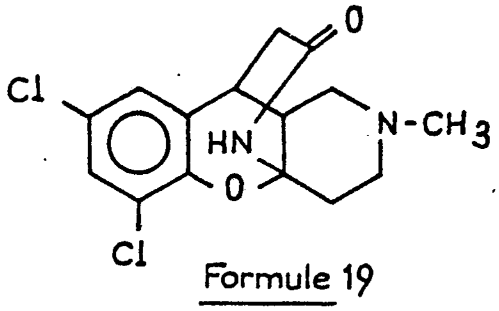

Formule 19

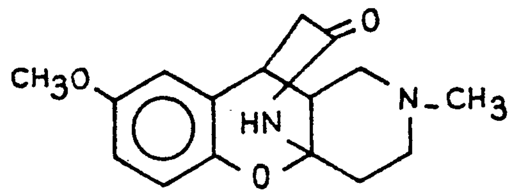

Formule 20

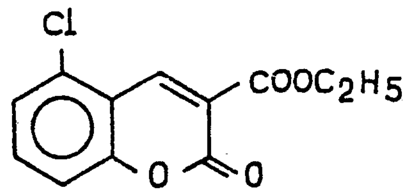

Formule 21

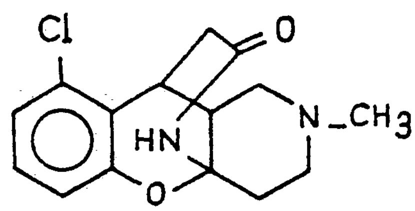

Formule 22

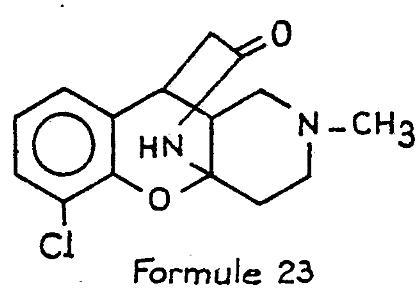

Formule 23

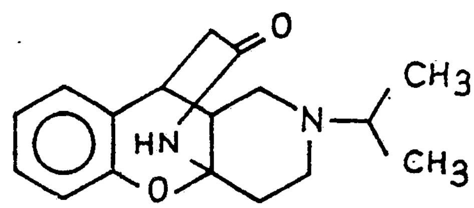

Formule 24

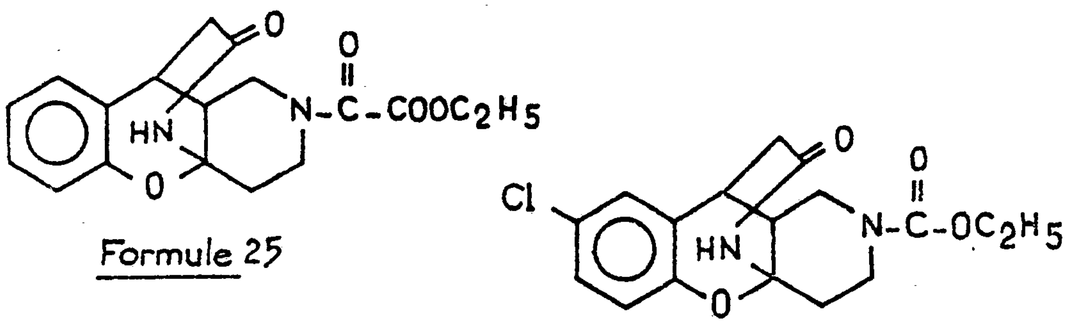

Formule 25

Formule 30

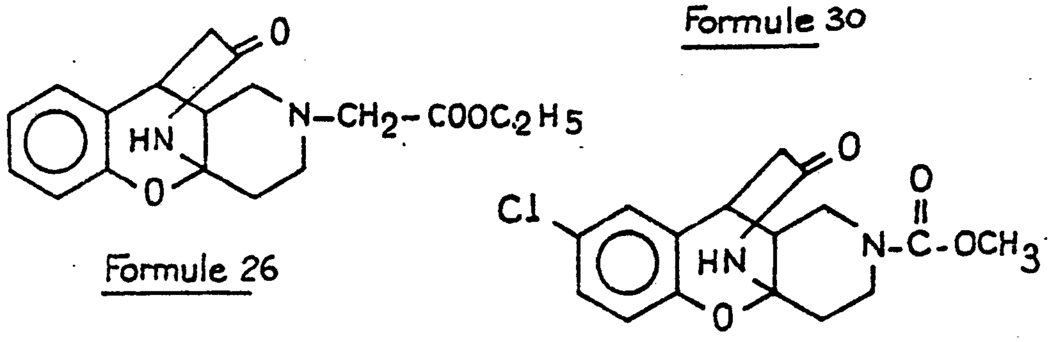

Formule 26

Formule 31

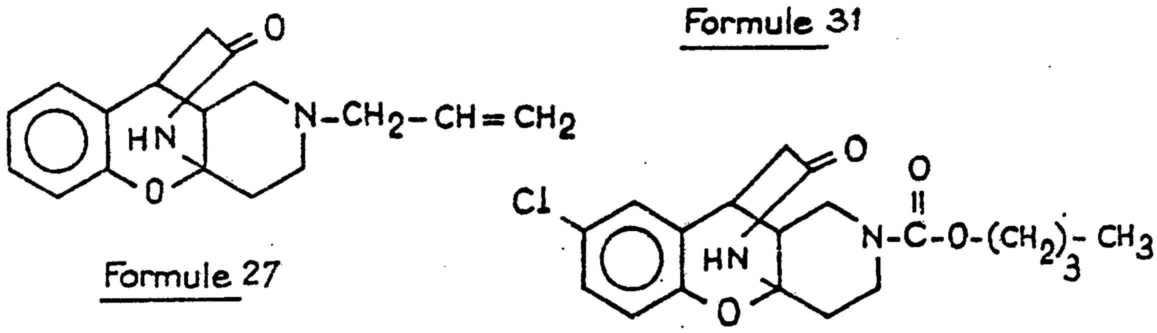

Formule 27

Formule 32

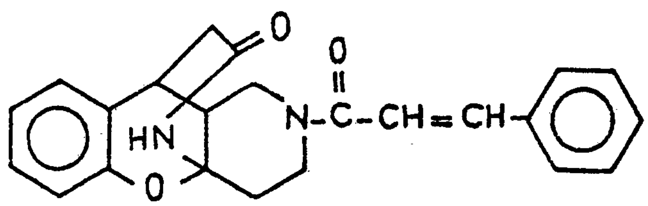

Formule 28

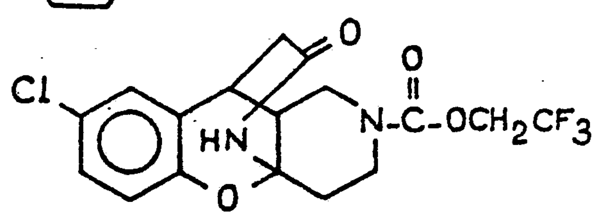

Formule 33

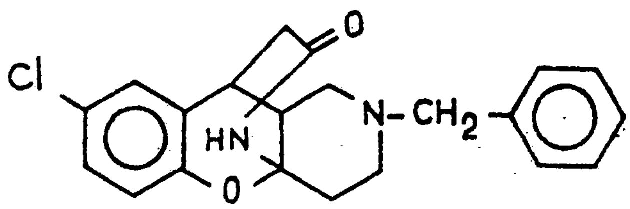

Formule 29

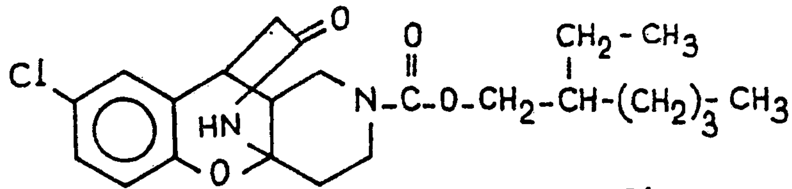

Formule 34

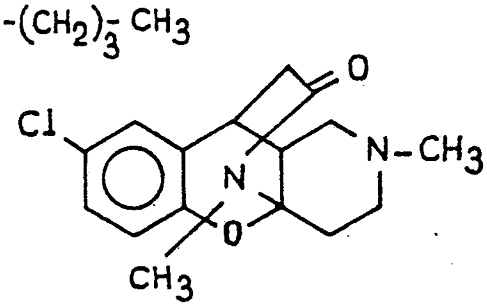

Formule 39

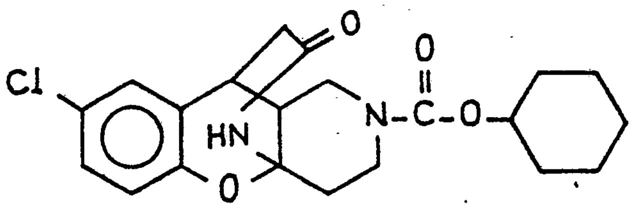

Formule 35

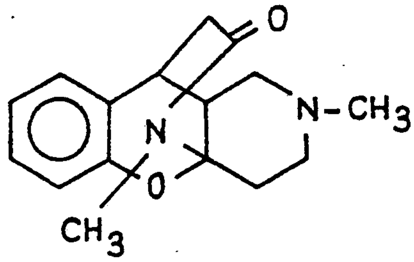

Formule 40

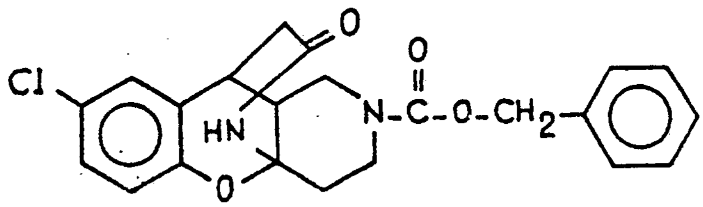

Formule 36

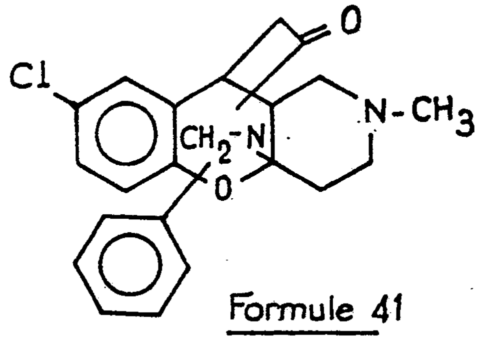

Formule 41

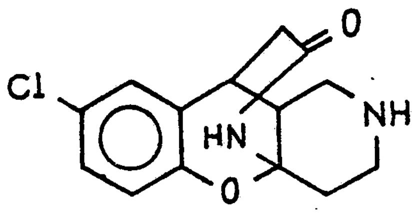

Formule 37

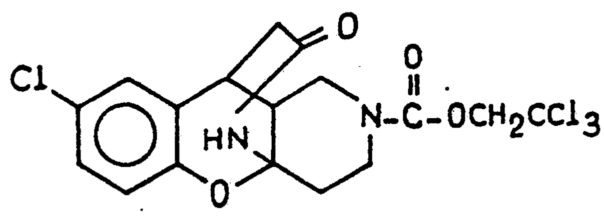

Formule 42

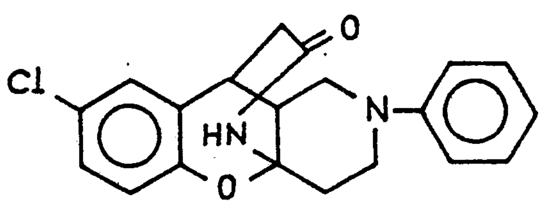

Formule 38

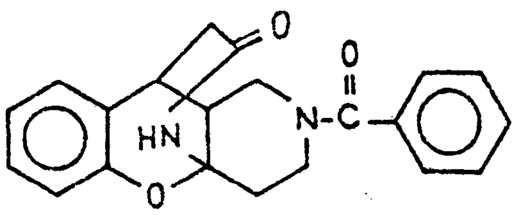

Formule 43

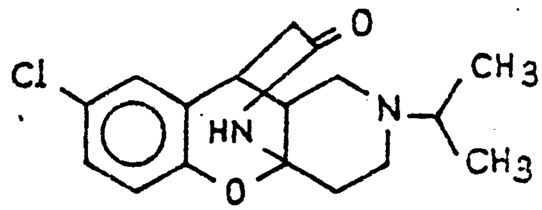

Formule 46

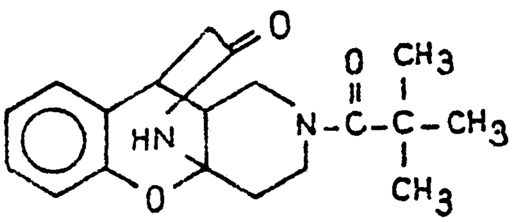

Formule 44

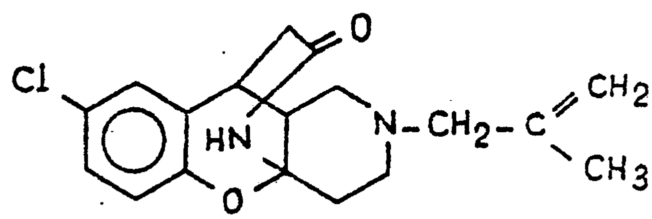

Formule 47

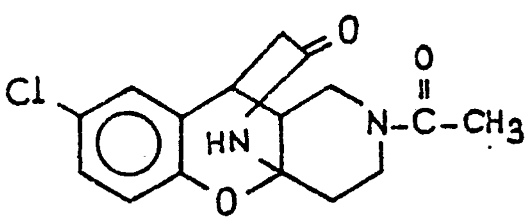

Formule 45